# EUROPEAN PATENT APPLICATION

(11) **EP 2 500 023 A1**
(43) Date of publication of application: **19.09.2012**
(21) Application number: 12171437.2
(22) Date of filing: 14.08.2007
(51) Int. Cl.: A61K 31/7036, A61K 47/18, A61K 47/12, A61K 47/02, A61K 47/04, A61K 9/08, A61K 9/19, A61P 31/04, A61P 31/00

(54) **Antibiotics composition comprising beta-lactam antibiotic, beta-lactamase inhibitor, and buffer component**

(30) Priority: 25.08.2006 CN 200610015438
(62) Divisional of application: 07800696.2
(71) Applicant: Tianjin Hemay Bio-Tech Co., Ltd., Tianjin 300457 (CN)
(72) Inventor: Zhang, Hesheng, 300457 Tianjin (CN)
(74) Representative: Hryszkiewicz, Danuta

(57) **Abstract**

A pharmaceutical composition, including: at least one β-lactam antibiotic, at least one β-lactamase inhibitor, and at least one buffer component. The composition can be formulated with at least one of aminoglycoside antibiotics into solution for controlling microbial infection in a container.

## Description

The invention relates to a pharmaceutical composition comprising at least one β-lactam antibiotic and at least one buffer component. The pharmaceutical composition optionally further comprises: (a) at least one β-lactamase inhibitor; (b) at least one aminoglycoside antibiotic; or (c) at least one β-lactamase inhibitor and at least one aminoglycoside antibiotic; mixed in a single container together with the β-lactam antibiotic and the buffer component. The pharmaceutical composition is useful as an anti-microbial drug.

β-Lactam antibiotics inhibit the synthesis of bacterial cell wall by inhibiting the activity of D-alanyl-D-alanine transpeptidase (peptidoglycan transpeptidase) inside bacteria. Peptidoglycans are linear polysaccharide polypeptides with a network structure alternately comprising N-acetyl-glucosamine (Glc-NAc) and Mur-NAc. The transpeptide cross-linking reaction of these linear polymers catalysed by peptidoglycan transpeptidase results in network architecture and completes cell wall synthesis. β-lactam antibiotics irreversibly inhibit the activity of the peptidoglycan transpeptidase and cause a failure of bacterial cell wall formation. Without cell wall, bacterial cells don't have a definite shape and sustain high permeation pressure inside cells, which causes bacteriolysis resulting in the death of bacteria. β-Lactam antibiotics are the most widely used antibiotics in clinical medicine.

Bacteria have subsequently evolved to produce β-lactamase, which can hydrolyze the amido bond of the β-lactam ring of β-lactam antibiotics and transform β-lactam antibiotics into metabolites lacking antibacterial activity. In 1976, it was discovered that clavulanic acid separated from the fermented fluid of rod-like streptomycete was a unique β-lactamase inhibitor. Soon thereafter, other β-lactamase inhibitors, especially sulbactam and its lipid prodrugs, i.e., composition of ampicillin sodium and sulbactam sodium, and tazobactam became widely used in clinical settings.

Another type of antibiotics widely used is aminoglycoside antibiotics. Aminoglycoside antibiotics are glycosides formed from aminosugar (monosaccharide or disaccharide) and amino-cyclitol. They are alkaline in nature owing to their amino and other basic functional groups. Due to their broad anti-bacterial spectrum, high anti-bacterial activity, frequent clinical use, there have been more than 20 species of aminoglycosides developed since the discovery of the first aminoglycoside antibiotic, streptomycin, which was isolated from Streptothrix in 1940.

The anti-bacterial mechanism of action of aminoglycoside antibiotics is entirely different from that of β-lactam antibiotics. After entering bacteria the aminoglycoside antibiotic conjugates with the 30S subunit protein, which causes errors when tRNA translates mRNA code and results in non-functioning proteins inhibiting cell growth.

It is generally known that the combination of β-lactam antibiotics with aminoglycoside antibiotics provides an anti-bacterial synergy. However, β-lactam antibiotics are acidic whereas aminoglycoside antibiotics are basic. When these two types of antibiotics are dissolved in the same solution, either a salt precipitates out due to acid-base reaction, or the amino group of the aminoglycoside antibiotic reacts with the β-lactam group of the β-lactam antibiotics. Both of the reactions drastically reduce the efficacy of these types of antibiotics. Therefore, mixing of these two types of antibiotics in the same solution is normally disadvantageous.

The development of a pharmaceutical composition, in which β-lactam antibiotics and aminoglycoside antibiotics could not only stably coexist without loss of efficacy but could also act in synergy, would bring about a breakthrough in the field of infectious diseases. The key to the development of such a composition is the discovery of a system, in which a mixture of β-lactam antibiotics and aminoglycoside antibiotics would not form a precipitate, and wherein a reaction between the β-lactam group of β-lactam antibiotics with the amino group of aminoglycoside antibiotics can be completely inhibited.

As a result of systematic studies, it has been found that when the pH value is controlled in the range of between 3 and 9, a precipitate resulting from a salt forming reaction and the reaction between the β-lactam group of β-lactam antibiotics with the amino group of aminoglycoside antibiotics can be inhibited to a certain degree; when the pH value is controlled in the range of between 4 and 8, the precipitate and the reaction between the amino group and the β-lactam group can be inhibited to a significant degree; and when the pH value is controlled in the range of between 6 and 7.5, the precipitate and the reaction between the amino group and the β-lactam group can be completely inhibited.

In view of the above-described problems, it is one objective of the invention to provide a pharmaceutical composition which can be used as an anti-microbial and anti-infection drug.

In order to achieve the above objectives, in accordance with one embodiment of the invention, provided is a pharmaceutical composition, comprising: at least one β-lactam antibiotic and at least one buffer component. A liquid formulation of the pharmaceutical composition is clear and transparent without turbidity or precipitate, and the efficacy of the β-lactam antibiotic is maintained for at least 8 hours.

In a class of this embodiment, the composition is provided as a mixture with at least an aminoglycoside antibiotic in the same container. The resultant solution is clear and transparent without turbidity or precipitate, and the efficacy of the β-lactam antibiotic is maintained for at least 8 hours.

In another class of this embodiment, a β-lactamase inhibitor is further added to the pharmaceutical composition, the resultant solution is clear and transparent without turbidity or precipitate, and the efficacy of the β-lactam antibiotic and the β-lactamase inhibitor in the composition is maintained for at least 8 hours.

In another class of this embodiment, a β-lactamase inhibitor and an aminoglycoside antibiotic are simultaneously added to the pharmaceutical composition, the resultant solution is clear and transparent without turbidity or precipitate, and the efficacy of the β-lactam antibiotic, β-lactamase inhibitor, and aminoglycoside antibiotic in the composition is maintained for at least 8 hours.

In another class of this embodiment, the β-lactam antibiotic is without limitation cefalothine, cefaloridine, cefazolin, cefapirin, cefaloglycin, cefalexin, cefadroxil, cefaclor, cefamandole, cefsulodine , cefoperazone, cefuroxime, cefotaxime, ceftizoxime, cefmenoxime, ceftriaxone, cefuzonam, cefixime, ceftazidime, ceftibuten, cefodizime, cephalosporin, cefpirome, cefepime, cefclidin, cefoxitin, cefmetazol, cefbuperazone, cefotetan, latamoxef, flomoxef, loracarbef, cefaloridine, latamoxef, cefminox, cefpiramide, cefonicid, ceforanide, cefacetrile, cefathiamidine, pheneticillin, propicillin, azidocillin, trityl penicillin, methicillin, nafcillin, oxacillin, cloxacillin, dicloxacillin, flucloxacillin, mecillinam, adicillin, ampicillin, amoxicillin, ticarcillin, carbenicillin, sulbenicillin, hetacillin, apalcillin, mezlocillin, temocillin, formidacillin, aspoxicillin, lenampicillin, azlocillin, pivampicillin, furbenicillin, phenoxymethypenicillin, apalcillin, nafcillin, metampicillin, or a pharmaceutically acceptable salt or hydrate thereof. Particularly, the β-lactam antibiotic is cefoperazone, ceftriaxone, cefodizime, or mezlocillin.

In another class of this embodiment, the aminoglycoside antibiotic is without limitation streptomycin, dibekacin, kanamycin, tobramycin, amikacin, arbekacin, gentamicin, sagamicin, Isepamicin, sisomicin, netilmicin, neomycin, paromomycin, etimicin, astromicin, ribostamycin, micronomicin, spectinomycin, or a pharmaceutically acceptable salt or hydrate thereof. Particularly, the aminoglycoside antibiotic is amikacin, gentamicin, or etimicin.

In another class of this embodiment, the β-lactamase inhibitor is without limitation clavulanic acid, sulbactam, tazobactam, a combimation composition of ampicillin sodium and sulbactam sodium, or a pharmaceutically acceptable salt or hydrate thereof. Particularly, the β-lactamase inhibitor is sulbactam, or tazobactam.

In another class of this embodiment, the buffer system is without limitation citric acid/citrate system or any other organic polyacid buffer system, phosphoric acid/phosphate system or any other inorganic acid buffer system, acetic acid/acetate system or any other organic monoacid system, arginine system or any other amino acid system, tris/HCl system, or any other pharmaceutically acceptable buffer system. Particularly, the buffer solution system is citric acid/citrate system, phosphoric acid/phosphate system, acetic acid/acetate system, arginine system, or carbonic acid/carbonate system. More particularly, the buffer system is citric acid/citrate system, phosphoric acid/phosphate system, or acetic acid/acetate system. The buffer component in the examples of this invention is sodium citrate.

In another class of this embodiment, the effective pH range of the buffer solution is between 4 and 8. Particularly, the effective pH range of the buffer solution is between 5.5 and 7.5. More particularly, the effective pH range of the buffer solution is between 6.0 and 6.75.

In another class of this embodiment, a concentration range of the buffer solution is between 1 and 500 mM; particularly, between 5 and 100 mM; and more particularly between 10 and 60 mM.

The pharmaceutical compositions described herein can be divided in four classes according to their clinical applications, as follows:

1) In the first class is a pharmaceutical composition, comprising: at least one β-lactam antibiotic, at least one β-lactamase inhibitor, at least one aminoglycoside antibiotic, and a buffer component. This pharmaceutical composition, formulated as an injectable solution, is clear and transparent without turbidity or precipitate, and the efficacy of the β-lactam antibiotic and the aminoglycoside antibiotic in this composition is maintained. A representative unit-dose composition in this class, comprises: between 0.1 g and 5 g of a β-lactam antibiotic, between 0.1 g and 5 g of a β-lactamase inhibitor, between 0.01 g and 5 g of sodium citrate, and between 0.01 g and 5 g of an aminoglycoside antibiotic. The pharmaceutical composition can be prepared as a solution preparation with freeze preservation, or a lyophilized injectable powder, which is dissolved prior to use.

2) In the second class is a pharmaceutical composition, comprising: at least one β-lactam antibiotic, at least one buffer component, and at least one aminoglycoside antibiotic. The working pH range of the buffer component is between 6 and 7. This pharmaceutical composition, formulated as an injectable solution, is clear and transparent without turbidity or precipitate, and the efficacy of the β-lactam antibiotic and the aminoglycoside antibiotic in this composition is maintained. A representative unit-dose composition in this class, comprises: between 0.1 g and 5 g of β-lactam antibiotic, between 0.01 g and 5 g of sodium citrate, and between 0.01 g and 5 g of an aminoglycoside antibiotic. The pharmaceutical composition can be prepared as a solution preparation stored with freeze preservation, or a lyophilized injectable powder, which is dissolved prior to use.

3) In the third class is a pharmaceutical composition, comprising: at least one β-lactam antibiotic, at least one β-lactamase inhibitor, and a buffer component. The working pH range of the buffer component is between 6 and 7. This pharmaceutical composition, formulated as an injectable solution, is clear and transparent without turbidity or precipitate. The efficacy of the β-lactam antibiotic and the aminoglycoside antibiotic in this composition is maintained when the two antibiotics are mixed together in a container. A representative unit-dose composition in this class, comprises: between 0.1 g and 5 g of β-lactam antibiotic, between 0.1 g and 5 g of β-lactamase inhibitor, and between 0.01 g and 5 g of sodium citrate. The pharmaceutical composition can be prepared as a solution preparation stored with freeze preservation, or a lyophilized injectable powder, which is dissolved prior to use.

4) In the fourth class is a pharmaceutical composition, comprising: at least one β-lactam antibiotic and at least one buffer component. When the pharmaceutical composition, formulated as an injectable solution, is mixed in a container with at least a solution of an aminoglycoside antibiotic, a clear and transparent mixture without turbidity or precipitate is obtained. The efficacy of the β-lactam antibiotic and the aminoglycoside antibiotic is maintained. A representative unit-dose composition in this class, comprises: between 0.1 g and 5 g of a β-lactam antibiotic, and between 0.01 g and 5 g of sodium citrate. The pharmaceutical composition can be prepared as a solution preparation stored with freeze preservation, or a lyophilized injectable powder, which is dissolved prior to use.

In clinical application of the pharmaceutical composition of the invention, salts or hydrates of β-lactam antibiotics are preferable.

In clinical application of the pharmaceutical composition of the invention, if β-lactamase inhibitor is needed, a salt of the β-lactamase inhibitor, such as potassium clavulanate, sulbactam sodium, tazobactam sodium or a hydrate thereof are preferable.

In clinical application of the pharmaceutical composition of the invention, the weight ratio of a β-lactam antibiotic to a β-lactamase inhibitor is preferably 1:1, 2:1, 4:1, or 8:1.

The pharmaceutical compositions described herein include, but are not limited, to the following representative unit dose Formulations:

Formulation 1: cefoperazone sodium 0.1-4 g, sulbactam sodium 0.1-4 g, amikacin sulfate 20-800 mg, sodium citrate 10-5000 mg, and citric acid 20-5000 mg.

Formulation 2: cefoperazone sodium 0.5-4 g, sulbactam sodium 0.1-4 g, 0.2-50 mg, gentamicin 20-800 mg, sodium citrate 10-5000 mg, and citric acid 20-1200 mg.

Formulation 3: cefoperazone sodium 0.5-4 g, sulbactam sodium 0.1-4 g, tobramycin 20-800 mg, sodium citrate 10-5000 mg, and citric acid 20-1200 mg.

Formulation 4: cefoperazone sodium 0.5-4 g, sulbactam sodium 0.1-4 g, etimicin 20-800 mg, sodium citrate 10-5000 mg, and citric acid 20-1200 mg.

Formulation 5: cefradine 0.5-4 g, amikacin sulfate 20-800 mg, sodium citrate 10-5000 mg, and citric acid 20-1200 mg.

Formulation 6: cefoperazone sodium 0.5-4 g, sulbactam sodium 0.1-4 g, dibekacin 20-800 mg, sodium citrate 10-5000 mg, and citric acid 20-1200 mg.

Formulation 7: cefoperazone sodium 0.5-4 g, sulbactam sodium 0.1-4 g, arbekacin 20-800 mg, sodium citrate 10-5000 mg, and citric acid 20-1200 mg.

Formulation 8: cefoperazone sodium 0.5-4 g, sulbactam sodium 0.1-4 g, kanamycin 20-800 mg, sodium citrate 10-5000 mg, and citric acid 20-1200 mg.

Formulation 9: cefoperazone sodium 0.5-4 g, sulbactam sodium 0.1-4 g, sagamicin 20-800 mg, sodium citrate 10-5000 mg, and citric acid 20-1200 mg.

Formulation 10: cefoperazone sodium 0.5-4 g, sulbactam sodium 0.1-4 g, lsepamicin 20-800 mg, sodium citrate 10-5000 mg, and citric acid 20-1200 mg.

Formulation 11: cefoperazone sodium 0.5-4 g, sulbactam sodium 0.1-4 g, neomycin 20-800 mg, sodium citrate 10-5000 mg, and citric acid 20-1200 mg.

Formulation 12: cefoperazone sodium 0.5-4 g, sulbactam sodium 0.1-4 g, paromomycin 20-800 mg, sodium citrate 10-5000 mg, and citric acid 20-1200 mg.

Formulation 13: cefoperazone sodium 0.5-4 g, sulbactam sodium 0.1-4 g, sisomicin 20-800 mg, sodium citrate 10-5000 mg, and citric acid 20-1200 mg.

Formulation 14: cefoperazone sodium 0.5-4 g, sulbactam sodium 0.1-4 g, netilmicin 20-800 mg, sodium citrate 10-5000 mg, and citric acid 20-1200 mg.

Formulation 15: cefoperazone sodium 0.5-4 g, tazobactam 0.1-4 g, amikacin sulfate 20-800 mg, sodium citrate 10-5000 mg, and citric acid 20-1200 mg.

Formulation 16: cefoperazone sodium 0.5-4 g, tazobactam 0.1-4 g, gentamicin 20-800 mg, sodium citrate 10-5000 mg, and citric acid 20-1200 mg.

Formulation 17: cefoperazone sodium 0.5-4 g, tazobactam 0.1-4 g, tobramycin 20-800 mg, sodium citrate 10-5000 mg, and citric acid 20-1200 mg.

Formulation 18: cefoperazone sodium 0.5-4 g, tazobactam 0.1-4 g, etimicin 20-800 mg, sodium citrate 10-5000 mg, and citric acid 20-1200 mg.

Formulation 19: cefoperazone sodium 0.5-4 g, tazobactam 0.1-4 g, dibekacin 20-800 mg, sodium citrate 10-5000 mg, and citric acid 20-1200 mg.

Formulation 20: cefoperazone sodium 0.5-4 g, tazobactam 0.1-4 g, arbekacin 20-800 mg, sodium citrate 10-5000 mg, and citric acid 20-1200 mg.

Formulation 21: cefoperazone sodium 0.5-4 g, tazobactam 0.1-4 g, kanamycin 20-800 mg, sodium citrate 10-5000 mg, and citric acid 20-1200 mg.

Formulation 22: cefoperazone sodium 0.5-4 g, tazobactam 0.1-4 g, sagamicin 20-800 mg, sodium citrate 10-5000 mg, and citric acid 20-1200 mg.

Formulation 23: cefoperazone sodium 0.5-4 g, tazobactam 0.1-4 g, lsepamicin 20-800 mg, sodium citrate 10-5000 mg, and citric acid 20-1200 mg.

Formulation 24: cefoperazone sodium 0.5-4 g, tazobactam 0.1-4 g, neomycin 20-800 mg, sodium citrate 10-5000 mg, and citric acid 20-1200 mg.

Formulation 25: cefoperazone sodium 0.5-4 g, tazobactam 0.1-4 g, paromomycin 20-800 mg, sodium citrate 10-5000 mg, and citric acid 20-1200 mg.

Formulation 26: cefoperazone sodium 0.5-4 g, tazobactam 0.1-4 g, sisomicin 20-800 mg, sodium citrate 10-5000 mg, and citric acid 20-1200 mg.

Formulation 27: cefoperazone sodium 0.5-4 g, tazobactam 0.1-4 g, netilmicin 20-800 mg, sodium citrate 10-5000 mg, and citric acid 20-1200 mg.

Formulation 28: cefodizime sodium 0.5-4 g, amikacin sulfate 20-800 mg, sodium citrate 10-5000 mg, and citric acid 20-1200 mg.

Formulation 29: cefodizime sodium 0.5-4 g, gentamicin 20-800 mg, sodium citrate 10-5000 mg, and citric acid 20-1200 mg.

Formulation 30: cefodizime sodium 0.5-4 g, tobramycin 20-800 mg, sodium citrate 10-5000 mg, and citric acid 20-1200 mg.

Formulation 31: cefodizime sodium 0.5-4 g, etimicin 20-800 mg, sodium citrate 10-5000 mg, and citric acid 20-1200 mg.

Formulation 32: cefodizime sodium 0.5-4 g, dibekacin 20-800 mg, sodium citrate 10-5000 mg, and citric acid 20-1200 mg.

Formulation 33: cefodizime sodium 0.5-4 g, arbekacin 20-800 mg, sodium citrate 10-5000 mg, and citric acid 20-1200 mg.

Formulation 34: cefodizime sodium 0.5-4 g, kanamycin 20-800 mg, sodium citrate 10-5000 mg, and citric acid 20-1200 mg.

Formulation 35: cefodizime sodium 0.5-4 g, sagamicin 20-800 mg, sodium citrate 10-5000 mg, and citric acid 20-1200 mg.

Formulation 36: cefodizime sodium 0.5-4 g, isepamicin 20-800 mg, sodium citrate 10-5000 mg, and citric acid 20-1200 mg.

Formulation 37: cefodizime sodium 0.5-4 g, neomycin 20-800 mg, sodium citrate 10-5000 mg, and citric acid 20-1200 mg.

Formulation 38: cefodizime sodium 0.5-4 g, paromomycin 20-800 mg, sodium citrate 10-5000 mg, and citric acid 20-1200 mg.

Formulation 39: cefodizime sodium 0.5-4 g, sisomicin 20-800 mg, sodium citrate 10-5000 mg, and citric acid 20-1200 mg.

Formulation 40: cefodizime sodium 0.5-4 g, netilmicin 20-800 mg, sodium citrate 10-5000 mg, and citric acid 20-1200 mg.

Formulation 41: cefoperazone sodium 0.5-4 g, potassium clavulanate 0.1-4 g, amikacin sulfate 20-800 mg, sodium citrate 10-5000 mg, and citric acid 20-1200 mg.

Formulation 42: cefoperazone sodium 0.5-4 g, potassium clavulanate 0.1-4 g, gentamicin 20-800 mg, sodium citrate 10-5000 mg, and citric acid 20-1200 mg.

Formulation 43: cefoperazone sodium 0.5-4 g, potassium clavulanate 0.1-4 g, tobramycin 20-800 mg, sodium citrate 10-5000 mg, and citric acid 20-1200 mg.

Formulation 44: cefoperazone sodium 0.5-4 g, potassium clavulanate 0.1-4 g, etimicin 20-800 mg, sodium citrate 10-5000 mg, and citric acid 20-1200 mg.

Formulation 45: cefoperazone sodium 0.5-4 g, potassium clavulanate 0.1-4 g, dibekacin 20-800 mg, sodium citrate 10-5000 mg, and citric acid 20-1200 mg.

Formulation 46: cefoperazone sodium 0.5-4 g, potassium clavulanate 0.1-4 g, arbekacin 20-800 mg, sodium citrate 10-5000 mg, and citric acid 20-1200 mg.

Formulation 47: cefoperazone sodium 0.5-4 g, potassium clavulanate 0.1-4 g, kanamycin 20-800 mg, sodium citrate 10-5000 mg, and citric acid 20-1200 mg.

Formulation 48: cefoperazone sodium 0.5-4 g, potassium clavulanate 0.1-4 g, sagamicin 20-800 mg, sodium citrate 10-5000 mg, and citric acid 20-1200 mg.

Formulation 49: cefoperazone sodium 0.5-4 g, potassium clavulanate 0.1-4 g, Isepamicin 20-800 mg, sodium citrate 10-5000 mg, and citric acid 20-1200 mg.

Formulation 50: cefoperazone sodium 0.5-4 g, potassium clavulanate 0.1-4 g, neomycin 20-800 mg, sodium citrate 10-5000 mg, and citric acid 20-1200 mg.

Formulation 51: cefoperazone sodium 0.5-4 g, potassium clavulanate 0.1-4 g, paromomycin 20-800 mg, sodium citrate 10-5000 mg, and citric acid 20-1200 mg.

Formulation 52: cefoperazone sodium 0.5-4 g, potassium clavulanate 0.1-4 g, sisomicin 20-800 mg, sodium citrate 10-5000 mg, and citric acid 20-1200 mg.

Formulation 53: ceftriaxone sodium 0.5-4 g, potassium clavulanate 0.1-4 g, netilmicin 20-800 mg, sodium citrate 10-5000 mg, and citric acid 20-1200 mg.

Formulation 54: ceftriaxone sodium 0.5-4 g, sulbactam sodium 0.1-4 g, amikacin sulfate 20-800 mg, sodium citrate 10-5000 mg, and citric acid 20-1200 mg.

Formulation 55: ceftriaxone sodium 0.5-4 g, sulbactam sodium 0.1-4 g, gentamicin 20-800 mg, sodium citrate 10-5000 mg, and citric acid 20-1200 mg.

Formulation 56: ceftriaxone sodium 0.5-4 g, sulbactam sodium 0.1-4 g, tobramycin 20-800 mg, sodium citrate 10-5000 mg, and citric acid 20-1200 mg.

Formulation 57: ceftriaxone sodium 0.5-4 g, sulbactam sodium 0.1-4 g, etimicin 20-800 mg, sodium citrate 10-5000 mg, and citric acid 20-1200 mg.

Formulation 58: ceftriaxone sodium 0.5-4 g, sulbactam sodium 0.1-4 g, dibekacin 20-800 mg, sodium citrate 10-5000 mg, and citric acid 20-1200 mg.

Formulation 59: ceftriaxone sodium 0.5-4 g, sulbactam sodium 0.1-4 g, arbekacin 20-800 mg, sodium citrate 10-5000 mg, and citric acid 20-1200 mg.

Formulation 60: ceftriaxone sodium 0.5-4 g, sulbactam sodium 0.1-4 g, kanamycin 20-800 mg, sodium citrate 10-5000 mg, and citric acid 20-1200 mg.

Formulation 61: ceftriaxone sodium 0.5-4 g, sulbactam sodium 0.1-4 g, sagamicin 20-800 mg, sodium citrate 10-5000 mg, and citric acid 20-1200 mg.

Formulation 62: ceftriaxone sodium 0.5-4 g, sulbactam sodium 0.1-4 g, lsepamicin 20-800 mg, sodium citrate 10-5000 mg, and citric acid 20-1200 mg.

Formulation 63: ceftriaxone sodium 0.5-4 g, sulbactam sodium 0.1-4 g, neomycin 20-800 mg, sodium citrate 10-5000 mg, and citric acid 20-1200 mg.

Formulation 64: ceftriaxone sodium 0.5-4 g, sulbactam sodium 0.1-4 g, paromomycin 20-800 mg, sodium citrate 10-5000 mg, and citric acid 20-1200 mg.

Formulation 65: ceftriaxone sodium 0.5-4 g, sulbactam sodium 0.1-4 g, sisomicin 20-800 mg, sodium citrate 10-5000 mg, and citric acid 20-1200 mg.

Formulation 66: ceftriaxone sodium 0.5-4 g, sulbactam sodium 0.1-4 g, netilmicin 20-800 mg, sodium citrate 10-5000 mg, and citric acid 20-1200 mg.

Formulation 67: ceftazidime sodium 0.5-4 g, amikacin sulfate 20-800 mg, sodium citrate 10-5000 mg, and citric acid 20-1200 mg.

Formulation 68: ceftazidime sodium 0.5-4 g, gentamicin 20-800 mg, sodium citrate 10-5000 mg, and citric acid 20-1200 mg.

Formulation 69: ceftazidime sodium 0.5-4 g, tobramycin 20-800 mg, sodium citrate 10-5000 mg, and citric acid 20-1200 mg.

Formulation 70: ceftazidime sodium 0.5-4 g, etimicin 20-800 mg, sodium citrate 10-5000 mg, and citric acid 20-1200 mg.

Formulation 71: ceftazidime sodium 0.5-4 g, dibekacin 20-800 mg, sodium citrate 10-5000 mg, and citric acid 20-1200 mg.

Formulation 72: ceftazidime sodium 0.5-4 g, arbekacin 20-800 mg, sodium citrate 10-5000 mg, and citric acid 20-1200 mg.

Formulation 73: ceftazidime sodium 0.5-4 g, kanamycin 20-800 mg, sodium citrate 10-5000 mg, and citric acid 20-1200 mg.

Formulation 74: ceftazidime sodium 0.5-4 g, sagamicin 20-800 mg, sodium citrate 10-5000 mg, and citric acid 20-1200 mg.

Formulation 75: ceftazidime sodium 0.5-4 g, lsepamicin 20-800 mg, sodium citrate 10-5000 mg, and citric acid 20-1200 mg.

Formulation 76: ceftazidime sodium 0.5-4 g, neomycin 20-800 mg, sodium citrate 10-5000 mg, and citric acid 20-1200 mg.

Formulation 77: ceftazidime sodium 0.5-4 g, paromomycin 20-800 mg, sodium citrate 10-5000 mg, and citric acid 20-1200 mg.

Formulation 78: ceftazidime sodium 0.5-4 g, sisomicin 20-800 mg, sodium citrate 10-5000 mg, and citric acid 20-1200 mg.

Formulation 79: ceftazidime sodium 0.5-4 g, netilmicin 20-800 mg, sodium citrate 10-5000 mg, and citric acid 20-1200 mg.

Formulation 80: cefradine 0.5-4 g, gentamicin 20-800 mg, sodium citrate 10-5000 mg, and citric acid 20-1200 mg.

Formulation 81: c cefradine 0.5-4 g, tobramycin 20-800 mg, sodium citrate 10-5000 mg, and citric acid 20-1200 mg.

Formulation 82: cefradine 0.5-4 g, etimicin 20-800 mg, sodium citrate 10-5000 mg, and citric acid 20-1200 mg.

Formulation 83: cefradine 0.5-4 g, dibekacin 20-800 mg, sodium citrate 10-5000 mg, and citric acid 20-1200 mg.

Formulation 84: cefradine 0.5-4 g, arbekacin 20-800 mg, sodium citrate 10-5000 mg, and citric acid 20-1200 mg.

Formulation 85: cefradine 0.5-4 g, kanamycin 20-800 mg, sodium citrate 10-5000 mg, and citric acid 20-1200 mg.

Formulation 86: cefradine 0.5-4 g, sagamicin 20-800 mg, sodium citrate 10-5000 mg, and citric acid 20-1200 mg.

Formulation 87: cefradine 0.5-4 g, lsepamicin 20-800 mg, sodium citrate 10-5000 mg, and citric acid 20-1200 mg.

Formulation 88: cefradine 0.5-4 g, neomycin 20-800 mg, sodium citrate 10-5000 mg, and citric acid 20-1200 mg.

Formulation 89: cefradine 0.5-4 g, paromomycin 20-800 mg, sodium citrate 10-5000 mg, and citric acid 20-1200 mg.

Formulation 90: cefradine 0.5-4 g, sisomicin 20-800 mg, sodium citrate 10-5000 mg, and citric acid 20-1200 mg.

Formulation 91: cefradine 0.5-4 g, netilmicin 20-800 mg, sodium citrate 10-5000 mg, and citric acid 20-1200 mg.

Formulation 92: cefoperazone sodium 0.5-4 g, sulbactam sodium 0.1-4 g, etimicin 20-800 mg, monosodium phosphate 0.1-2 g, disodium phosphate 0.5-4 g.

Formulation 93: cefoperazone sodium 0.5-4 g, tazobactam sodium 0.1-4 g, etimicin 20-800 mg, monosodium phosphate 0.1-2 g, disodium phosphate 0.5-4 g.

Formulation 94: cefoperazone sodium 0.5-4 g, sulbactam sodium 0.1-4 g, gentamicin 20-800 mg, monosodium phosphate 0.1-2 g, disodium phosphate 0.5-4 g.

Formulation 95: cefoperazone sodium 0.5-4 g, tazobactam sodium 0.1-4 g, gentamicin 20-800 mg, sodium acetate 10-5000 mg, acetic acid 100-1200 mg.

Formulation 96: cefoperazone sodium 0.5-4 g, sulbactam 0.1-4 g, gentamicin 20-800 mg, arginine 10-5000 mg.

Formulation 97: cefoperazone 0.5-4 g, tazobactam 0.1-4 g, gentamicin 20-800 mg, arginine 10-5000 mg.

Formulation 98: cefoperazone 0.5-4 g, sulbactam 0.1-4 g, arginine 10-5000 mg.

Formulation 99: cefoperazone 0.5-4 g, sulbactam 0.1-4 g, gentamicin 20-800 mg, arginine 10-2000 mg.

Formulation 100: cefoperazone 0.5-4 g, arginine 100-5000 mg.

Formulation 101: cefbuperazone sodium 0.5-4 g, sulbactam sodium 0.1-4 g, sodium citrate 10-5000 mg, and citric acid 20-1200 mg.

Formulation 102: cefotetan sodium 0.5-4 g, sulbactam sodium 0.1-4 g, sodium citrate 10-5000 mg, and citric acid 20-1200 mg.

Formulation 103: latamoxef sodium 0.5-4 g, sulbactam sodium 0.1-4 g, sodium citrate 10-5000 mg, and citric acid 20-1200 mg.

Formulation 104: flomoxef sodium 0.5-4 g, sulbactam sodium 0.1-4 g, sodium citrate 10-5000 mg, and citric acid 20-1200 mg.

Formulation 105: loracarbef sodium 0.5-4 g, sulbactam sodium 0.1-4 g, sodium citrate 10-5000 mg, and citric acid 20-1200 mg.

Formulation 106: cefmetazol sodium 0.5-4 g, sulbactam sodium 0.1-4 g, sodium citrate 10-5000 mg, and citric acid 20-1200 mg.

Formulation 107: cefoxitin sodium 0.5-4 g, sulbactam sodium 0.1-4 g, sodium citrate 10-5000 mg, and citric acid 20-1200 mg.

Formulation 108: cefclidin sodium 0.5-4 g, sulbactam sodium 0.1-4 g, sodium citrate 10-5000 mg, and citric acid 20-1200 mg.

Formulation 109: cefpirome sodium 0.5-4 g, sulbactam sodium 0.1-4 g, sodium citrate 10-5000 mg, and citric acid 20-1200 mg.

Formulation 110: cephalosporin sodium 0.5-4 g, sulbactam sodium 0.1-4 g, sodium citrate 10-5000 mg, and citric acid 20-1200 mg.

Formulation 111: cefodizime sodium 0.5-4 g, sulbactam sodium 0.1-4 g, sodium citrate 10-5000 mg, and citric acid 20-1200 mg.

Formulation 112: ceftibuten sodium 0.5-4 g, sulbactam sodium 0.1-4 g, sodium citrate 10-5000 mg, and citric acid 20-1200 mg.

Formulation 113: ceftazidime sodium 0.5-4 g, sulbactam sodium 0.1-4 g, sodium citrate 10-5000 mg, and citric acid 20-1200 mg.

Formulation 114: cefixime sodium 0.5-4 g, sulbactam sodium 0.1-4 g, sodium citrate 10-5000 mg, and citric acid 20-1200 mg.

Formulation 115: cefuzonam sodium 0.5-4 g, sulbactam sodium 0.1-4 g, sodium citrate 10-5000 mg, and citric acid 20-1200 mg.

Formulation 116: ceftriaxone sodium 0.5-4 g, sulbactam sodium 0.1-4 g, sodium citrate 10-5000 mg, and citric acid 20-1200 mg.

Formulation 117: ceftizoxime sodium 0.5-4 g, sulbactam sodium 0.1-4 g, sodium citrate 10-5000 mg, and citric acid 20-1200 mg.

Formulation 118: cefmenoxime sodium 0.5-4 g, sulbactam sodium 0.1-4 g, sodium citrate 10-5000 mg, and citric acid 20-1200 mg.

Formulation 119: ceftizoxime sodium 0.5-4 g, sulbactam sodium 0.1-4 g, sodium citrate 10-5000 mg, and citric acid 20-1200 mg.

Formulation 120: cefotaxime sodium 0.5-4 g, sulbactam sodium 0.1-4 g, sodium citrate 10-5000 mg, and citric acid 20-1200 mg.

Formulation 121: cefuroxime sodium 0.5-4 g, sulbactam sodium 0.1-4 g, sodium citrate 10-5000 mg, and citric acid 20-1200 mg.

Formulation 122: cefoperazone sodium 0.5-4 g, sulbactam sodium 0.1-4 g, sodium citrate 10-5000 mg, and citric acid 20-1200 mg.

Formulation 123: cefsulodin sodium 0.5-4 g, sulbactam sodium 0.1-4 g, sodium citrate 10-5000 mg, and citric acid 20-1200 mg.

Formulation 124: cefamandole sodium 0.5-4 g, sulbactam sodium 0.1-4 g, sodium citrate 10-5000 mg, and citric acid 20-1200 mg.

Formulation 125: cefadroxil sodium 0.5-4 g, sulbactam sodium 0.1-4 g, sodium citrate 10-5000 mg, and citric acid 20-1200 mg.

Formulation 126: cefaclor sodium 0.5-4 g, sulbactam sodium 0.1-4 g, sodium citrate 10-5000 mg, and citric acid 20-1200 mg.

Formulation 127: cefalexin sodium 0.5-4 g, sulbactam sodium 0.1-4 g, sodium citrate 10-5000 mg, and citric acid 20-1200 mg.

Formulation 128: cefbuperazone sodium 0.5-4 g, tazobactam sodium 0.1-4 g, sodium citrate 10-5000 mg, and citric acid 20-1200 mg.

Formulation 129: cefotetan sodium 0.5-4 g, tazobactam sodium 0.1-4 g, sodium citrate 10-5000 mg, and citric acid 20-1200 mg.

Formulation 130: latamoxef sodium 0.5-4 g, tazobactam sodium 0.1-4 g, sodium citrate 10-5000 mg, and citric acid 20-1200 mg.

Formulation 131: flomoxef sodium 0.5-4 g, tazobactam sodium 0.1-4 g, sodium citrate 10-5000 mg, and citric acid 20-1200 mg.

Formulation 132: loracarbef sodium 0.5-4 g, tazobactam sodium 0.1-4 g, sodium citrate 10-5000 mg, and citric acid 20-1200 mg.

Formulation 133: cefmetazol sodium 0.5-4 g, tazobactam sodium 0.1-4 g, sodium citrate 10-5000 mg, and citric acid 20-1200 mg.

Formulation 134: cefoxitin sodium 0.5-4 g, tazobactam sodium 0.1-4 g, sodium citrate 10-5000 mg, and citric acid 20-1200 mg.

Formulation 135: cefclidin sodium 0.5-4 g, tazobactam sodium 0.1-4 g, sodium citrate 10-5000 mg, and citric acid 20-1200 mg.

Formulation 136: cefpirome sodium 0.5-4 g, tazobactam sodium 0.1-4 g, sodium citrate 10-5000 mg, and citric acid 20-1200 mg.

Formulation 137: cephalosporin sodium 0.5-4 g, tazobactam sodium 0.1-4 g, sodium citrate 10-5000 mg, and citric acid 20-1200 mg.

Formulation 138: cefodizime sodium 0.5-4 g, tazobactam sodium 0.1-4 g, sodium citrate 10-5000 mg, and citric acid 20-1200 mg.

Formulation 139: ceftibuten sodium 0.5-4 g, tazobactam sodium 0.1-4 g, sodium citrate 10-5000 mg, and citric acid 20-1200 mg.

Formulation 140: ceftazidime sodium 0.5-4 g, tazobactam sodium 0.1-4 g, sodium citrate 10-5000 mg, and citric acid 20-1200 mg.

Formulation 141: cefixime sodium 0.5-4 g, tazobactam sodium 0.1-4 g, sodium citrate 10-5000 mg, and citric acid 20-1200 mg.

Formulation 142: cefuzonam sodium 0.5-4 g, tazobactam sodium 0.1-4 g, sodium citrate 10-5000 mg, and citric acid 20-1200 mg.

Formulation 143: ceftriaxone sodium 0.5-4 g, tazobactam sodium 0.1-4 g, sodium citrate 10-5000 mg, and citric acid 20-1200 mg.

Formulation 144: ceftizoxime sodium 0.5-4 g, tazobactam sodium 0.1-4 g, sodium citrate 10-5000 mg, and citric acid 20-1200 mg.

Formulation 145: cefmenoxime sodium 0.5-4 g, tazobactam sodium 0.1-4 g, sodium citrate 10-5000 mg, and citric acid 20-1200 mg.

Formulation 146: ceftizoxime sodium 0.5-4 g, tazobactam sodium 0.1-4 g, sodium citrate 10-5000 mg, and citric acid 20-1200 mg.

Formulation 147: cefotaxime sodium 0.5-4 g, tazobactam sodium 0.1-4 g, sodium citrate 10-5000 mg, and citric acid 20-1200 mg.

Formulation 148: cefuroxime sodium 0.5-4 g, tazobactam sodium 0.1-4 g, sodium citrate 10-5000 mg, and citric acid 20-1200 mg.

Formulation 149: cefoperazone sodium 0.5-4 g, tazobactam sodium 0.1-4 g, sodium citrate 10-5000 mg, and citric acid 20-1200 mg.

Formulation 150: cefsulodin sodium 0.5-4 g, tazobactam sodium 0.1-4 g, sodium citrate 10-5000 mg, and citric acid 20-1200 mg.

Formulation 151: cefamandole sodium 0.5-4 g, tazobactam sodium 0.1-4 g, sodium citrate 10-5000 mg, and citric acid 20-1200 mg.

Formulation 152: cefadroxil sodium 0.5-4 g, tazobactam sodium 0.1-4 g, sodium citrate 10-5000 mg, and citric acid 20-1200 mg.

Formulation 153: cefaclor sodium 0.5-4 g, tazobactam sodium 0.1-4 g, sodium citrate 10-5000 mg, and citric acid 20-1200 mg.

Formulation 154: cefalexin sodium 0.5-4 g, tazobactam sodium 0.1-4 g, sodium citrate 10-5000 mg, and citric acid 20-1200 mg.

Formulation 155: cefbuperazone sodium 0.5-4 g, sodium citrate 10-5000 mg, and citric acid 20-1200 mg.

Formulation 156: cefotetan sodium 0.5-4 g, sodium citrate 10-5000 mg, and citric acid 20-1200 mg.

Formulation 157: latamoxef sodium 0.5-4 g, sodium citrate 10-5000 mg, and citric acid 20-1200 mg.

Formulation 158: flomoxef sodium 0.5-4 g, sodium citrate 10-5000 mg, and citric acid 20-1200 mg.

Formulation 159: loracarbef sodium 0.5-4 g, sodium citrate 10-5000 mg, and citric acid 20-1200 mg.

Formulation 160: cefmetazol sodium 0.5-4 g, sodium citrate 10-5000 mg, and citric acid 20-1200 mg.

Formulation 161: cefoxitin sodium 0.5-4 g, sodium citrate 10-5000 mg, and citric acid 20-1200 mg.

Formulation 162: cefclidin sodium 0.5-4 g, sodium citrate 10-5000 mg, and citric acid 20-1200 mg.

Formulation 163: cefpirome sodium 0.5-4 g, sodium citrate 10-5000 mg, and citric acid 20-1200 mg.

Formulation 164: cephalosporin sodium 0.5-4 g, sodium citrate 10-5000 mg, and citric acid 20-1200 mg.

Formulation 165: cefodizime sodium 0.5-4 g, sodium citrate 10-5000 mg, and citric acid 20-1200 mg.

Formulation 166: ceftibuten sodium 0.5-4 g, sodium citrate 10-5000 mg, and citric acid 20-1200 mg.

Formulation 167: ceftazidime sodium 0.5-4 g, sodium citrate 10-5000 mg, and citric acid 20-1200 mg.

Formulation 168: cefixime sodium 0.5-4 g, sodium citrate 10-5000 mg, and citric acid 20-1200 mg.

Formulation 169: cefuzonam sodium 0.5-4 g, sodium citrate 10-5000 mg, and citric acid 20-1200 mg.

Formulation 170: ceftriaxone sodium 0.5-4 g, sodium citrate 10-5000 mg, and citric acid 20-1200 mg.

Formulation 171: ceftizoxime sodium 0.5-4 g, sodium citrate 10-5000 mg, and citric acid 20-1200 mg.

Formulation 172: cefmenoxime sodium 0.5-4 g, sodium citrate 10-5000 mg, and citric acid 20-1200 mg.

Formulation 173: ceftizoxime sodium 0.5-4 g, sodium citrate 10-5000 mg, and citric acid 20-1200 mg.

Formulation 174: cefotaxime sodium 0.5-4 g, sodium citrate 10-5000 mg, and citric acid 20-1200 mg.

Formulation 175: cefuroxime sodium 0.5-4 g, sodium citrate 10-5000 mg, and citric acid 20-1200 mg.

Formulation 176: cefoperazone sodium 0.5-4 g, sodium citrate 10-5000 mg, and citric acid 20-1200 mg.

Formulation 177: cefsulodin sodium 0.5-4 g, sodium citrate 10-5000 mg, and citric acid 20-1200 mg.

Formulation 178: cefamandole sodium 0.5-4 g, sodium citrate 10-5000 mg, and citric acid 20-1200 mg.

Formulation 179: cefadroxil sodium 0.5-4 g, sodium citrate 10-5000 mg, and citric acid 20-1200 mg.

Formulation 180: cefaclor sodium 0.5-4 g, sodium citrate 10-5000 mg, and citric acid 20-1200 mg.

Formulation 181: cefalexin sodium 0.5-4 g, sodium citrate 10-5000 mg, and citric acid 20-1200 mg.

Formulation 182: cefaloglycin sodium 0.5-4 g, sodium citrate 10-5000 mg, and citric acid 20-1200 mg.

Formulation 183: cefapirin sodium 0.5-4 g, sodium citrate 10-5000 mg, and citric acid 20-1200 mg.

Formulation 184: cefazolin sodium 0.5-4 g, sodium citrate 10-5000 mg, and citric acid 20-1200 mg.

Formulation 185: cefalothin sodium 0.5-4 g, sodium citrate 10-5000 mg, and citric acid 20-1200 mg.

Formulation 186: cefradine 0.5-4 g, sodium citrate 10-5000 mg, and citric acid 20-1200 mg.

Formulation 187: pheneticillin sodium 0.5-4 g, sodium citrate 10-5000 mg, and citric acid 20-1200 mg.

Formulation 188: propicillin sodium 0.5-4 g, sodium citrate 10-5000 mg, and citric acid 20-1200 mg.

Formulation 189: azidocillin sodium 0.5-4 g, sodium citrate 10-5000 mg, and citric acid 20-1200 mg.

Formulation 190: trityl penicillin sodium 0.5-4 g, sodium citrate 10-5000 mg, and citric acid 20-1200 mg.

Formulation 191: methicillin sodium 0.5-4 g, sodium citrate 10-5000 mg, and citric acid 20-1200 mg.

Formulation 192: nafcillin sodium 0.5-4 g, sodium citrate 10-5000 mg, and citric acid 20-1200 mg.

Formulation 193: oxacillin sodium 0.5-4 g, sodium citrate 10-5000 mg, and citric acid 20-1200 mg.

Formulation 194: cloxacillin sodium 0.5-4 g, sodium citrate 10-5000 mg, and citric acid 20-1200 mg.

Formulation 195: dicloxacillin sodium 0.5-4 g, sodium citrate 10-5000 mg, and citric acid 20-1200 mg.

Formulation 196: flucloxacillin sodium 0.5-4 g, sodium citrate 10-5000 mg, and citric acid 20-1200 mg.

Formulation 197: mecillinam sodium 0.5-4 g, sodium citrate 10-5000 mg, and citric acid 20-1200 mg.

Formulation 198: adicillin sodium 0.5-4 g, sodium citrate 10-2000 mg, and citric acid 20-1200 mg.

Formulation 199: ampicillin sodium 0.5-4 g, sodium citrate 10-5000 mg, and citric acid 20-1200 mg.

Formulation 200: amoxicillin sodium 0.5-4 g, sodium citrate 10-5000 mg, and citric acid 20-1200 mg.

Formulation 201: ticarcillin sodium 0.5-4 g, sodium citrate 10-5000 mg, and citric acid 20-1200 mg.

Formulation 202: carbenicillin sodium 0.5-4 g, sodium citrate 10-5000 mg, and citric acid 20-1200 mg.

Formulation 203: sulbenicillin sodium 0.5-4 g, sodium citrate 10-5000 mg, and citric acid 20-1200 mg.

Formulation 204: hetacillin sodium 0.5-4 g, sodium citrate 10-5000 mg, and citric acid 20-1200 mg.

Formulation 205: apalcillin sodium 0.5-4 g, sodium citrate 10-5000 mg, and citric acid 20-1200 mg.

Formulation 206: mezlocillin sodium 0.5-4 g, sodium citrate 10-5000 mg, and citric acid 20-1200 mg.

Formulation 207: temocillin sodium 0.5-4 g, sodium citrate 10-5000 mg, and citric acid 20-1200 mg.

Formulation 208: formidacillin sodium 0.5-4 g, sodium citrate 10-5000 mg, and citric acid 20-1200 mg.

Formulation 209: aspoxicillin sodium 0.5-4 g, sodium citrate 10-5000 mg, and citric acid 20-1200 mg.

Formulation 210: lenampicillin sodium 0.5-4 g, sodium citrate 10-5000 mg, and citric acid 20-1200 mg.

Formulation 211: pheneticillin sodium 0.5-4 g, sulbactam sodium 0.05-5 g, sodium citrate 10-5000 mg, and citric acid 20-1200 mg.

Formulation 212: propicillin sodium 0.5-4 g, sulbactam sodium 0.05-5 g, sodium citrate 10-5000 mg, and citric acid 20-1200 mg.

Formulation 213: azidocillin sodium 0.5-4 g, sulbactam sodium 0.05-5 g, sodium citrate 10-5000 mg, and citric acid 20-1200 mg.

Formulation 214: trityl penicillin sodium 0.5-4 g, sulbactam sodium 0.05-5 g, sodium citrate 10-5000 mg, and citric acid 20-1200 mg.

Formulation 215: methicillin sodium 0.5-4 g, sulbactam sodium 0.05-5 g, sodium citrate 10-5000 mg, and citric acid 20-1200 mg.

Formulation 216: nafcillin sodium 0.5-4 g, sulbactam sodium 0.05-5 g, sodium citrate 10-5000 mg, and citric acid 20-1200 mg.

Formulation 217: oxacillin sodium 0.5-4 g, sulbactam sodium 0.05-5 g, sodium citrate 10-5000 mg, and citric acid 20-1200 mg.

Formulation 218: cloxacillin sodium 0.5-4 g, sulbactam sodium 0.05-5 g, sodium citrate 10-5000 mg, and citric acid 20-1200 mg.

Formulation 219: dicloxacillin sodium 0.5-4 g, sulbactam sodium 0.05-5 g, sodium citrate 10-5000 mg, and citric acid 20-1200 mg.

Formulation 220: flucloxacillin sodium 0.5-4 g, sulbactam sodium 0.05-5 g, sodium citrate 10-5000 mg, and citric acid 20-1200 mg.

Formulation 221: mecillinam sodium 0.5-4 g, sulbactam sodium 0.05-5 g, sodium citrate 10-5000 mg, and citric acid 20-1200 mg.

Formulation 222: adicillin sodium 0.5-4 g, sulbactam sodium 0.05-5 g, sodium citrate 10-2000 mg, and citric acid 20-1200 mg.

Formulation 223: ampicillin sodium 0.5-4 g, sulbactam sodium 0.05-5 g, sodium citrate 10-5000 mg, and citric acid 20-1200 mg.

Formulation 224: amoxicillin sodium 0.5-4 g, sulbactam sodium 0.05-5 g, sodium citrate 10-5000 mg, and citric acid 20-1200 mg.

Formulation 225: ticarcillin sodium 0.5-4 g, sulbactam sodium 0.05-5 g, sodium citrate 10-5000 mg, and citric acid 20-1200 mg.

Formulation 226: carbenicillin sodium 0.5-4 g, sulbactam sodium 0.05-5 g, sodium citrate 10-5000 mg, and citric acid 20-1200 mg.

Formulation 227: sulbenicillin sodium 0.5-4 g, sulbactam sodium 0.05-5 g, sodium citrate 10-5000 mg, and citric acid 20-1200 mg.

Formulation 228: hetacillin sodium 0.5-4 g, sulbactam sodium 0.05-5 g, sodium citrate 10-5000 mg, and citric acid 20-1200 mg.

Formulation 229: apalcillin sodium 0.5-4 g, sulbactam sodium 0.05-5 g, sodium citrate 10-5000 mg, and citric acid 20-1200 mg.

Formulation 230: mezlocillin sodium 0.5-4 g, sulbactam sodium 0.05-5 g, sodium citrate 10-5000 mg, and citric acid 20-1200 mg.

Formulation 231: temocillin sodium 0.5-4 g, sulbactam sodium 0.05-5 g, sodium citrate 10-5000 mg, and citric acid 20-1200 mg.

Formulation 232: formidacillin sodium 0.5-4 g, sulbactam sodium 0.05-5 g, sodium citrate 10-5000 mg, and citric acid 20-1200 mg.

Formulation 233: aspoxicillin sodium 0.5-4 g, sulbactam sodium 0.05-5 g, sodium citrate 10-5000 mg, and citric acid 20-1200 mg.

Formulation 234: lenampicillin sodium 0.5-4 g, sulbactam sodium 0.05-5 g, sodium citrate 10-5000 mg, and citric acid 20-1200 mg.

Formulation 235: pheneticillin sodium 0.5-4 g, tazobactam sodium 0.05-5 g, sodium citrate 10-5000 mg, and citric acid 20-1200 mg.

Formulation 236: propicillin sodium 0.5-4 g, tazobactam sodium 0.05-5 g, sodium citrate 10-5000 mg, and citric acid 20-1200 mg.

Formulation 237: azidocillin sodium 0.5-4 g, tazobactam sodium 0.05-5 g, sodium citrate 10-5000 mg, and citric acid 20-1200 mg.

Formulation 238: trityl penicillin sodium 0.5-4 g, tazobactam sodium 0.05-5 g, sodium citrate 10-5000 mg, and citric acid 20-1200 mg.

Formulation 239: methicillin sodium 0.5-4 g, tazobactam sodium 0.05-5 g, sodium citrate 10-5000 mg, and citric acid 20-1200 mg.

Formulation 240: nafcillin sodium 0.5-4 g, tazobactam sodium 0.05-5 g, sodium citrate 10-5000 mg, and citric acid 20-1200 mg.

Formulation 241: oxacillin sodium 0.5-4 g, tazobactam sodium 0.05-5 g, sodium citrate 10-5000 mg, and citric acid 20-1200 mg.

Formulation 242: cloxacillin sodium 0.5-4 g, tazobactam sodium 0.05-5 g, sodium citrate 10-5000 mg, and citric acid 20-1200 mg.

Formulation 243: dicloxacillin sodium 0.5-4 g, tazobactam sodium 0.05-5 g, sodium citrate 10-5000 mg, and citric acid 20-1200 mg.

Formulation 244: flucloxacillin sodium 0.5-4 g, tazobactam sodium 0.05-5 g, sodium citrate 10-5000 mg, and citric acid 20-1200 mg.

Formulation 245: mecillinam sodium 0.5-4 g, tazobactam sodium 0.05-5 g, sodium citrate 10-5000 mg, and citric acid 20-1200 mg.

Formulation 246: adicillin sodium 0.5-4 g, tazobactam sodium 0.05-5 g, sodium citrate 10-2000 mg, and citric acid 20-1200 mg.

Formulation 247: ampicillin sodium 0.5-4 g, tazobactam sodium 0.05-5 g, sodium citrate 10-5000 mg, and citric acid 20-1200 mg.

Formulation 248: amoxicillin sodium 0.5-4 g, tazobactam sodium 0.05-5 g, sodium citrate 10-5000 mg, and citric acid 20-1200 mg.

Formulation 249: ticarcillin sodium 0.5-4 g, tazobactam sodium 0.05-5 g, sodium citrate 10-5000 mg, and citric acid 20-1200 mg.

Formulation 250: carbenicillin sodium 0.5-4 g, tazobactam sodium 0.05-5 g, sodium citrate 10-5000 mg, and citric acid 20-1200 mg.

Formulation 251: sulbenicillin sodium 0.5-4 g, tazobactam sodium 0.05-5 g, sodium citrate 10-5000 mg, and citric acid 20-1200 mg.

Formulation 252: hetacillin sodium 0.5-4 g, tazobactam sodium 0.05-5 g, sodium citrate 10-5000 mg, and citric acid 20-1200 mg.

Formulation 253: apalcillin sodium 0.5-4 g, tazobactam sodium 0.05-5 g, sodium citrate 10-5000 mg, and citric acid 20-1200 mg.

Formulation 254: mezlocillin sodium 0.5-4 g, tazobactam sodium 0.05-5 g, sodium citrate 10-5000 mg, and citric acid 20-1200 mg.

Formulation 255: temocillin sodium 0.5-4 g, tazobactam sodium 0.05-5 g, sodium citrate 10-5000 mg, and citric acid 20-1200 mg.

Formulation 256: formidacillin sodium 0.5-4 g, tazobactam sodium 0.05-5 g, sodium citrate 10-5000 mg, and citric acid 20-1200 mg.

Formulation 257: aspoxicillin sodium 0.5-4 g, tazobactam sodium 0.05-5 g, sodium citrate 10-5000 mg, and citric acid 20-1200 mg.

Formulation 258: lenampicillin sodium 0.5-4 g, tazobactam sodium 0.05-5 g, sodium citrate 10-5000 mg, and citric acid 20-1200 mg.

Formulation 259: pheneticillin sodium 0.5-4 g, potassium clavulanate 0.05-5 g, sodium citrate 10-5000 mg, and citric acid 20-1200 mg.

Formulation 260: propicillin sodium 0.5-4 g, potassium clavulanate 0.05-5 g, sodium citrate 10-5000 mg, and citric acid 20-1200 mg.

Formulation 267: azidocillin sodium 0.5-4 g, potassium clavulanate 0.05-5 g, sodium citrate 10-5000 mg, and citric acid 20-1200 mg.

Formulation 268: trityl penicillin sodium 0.5-4 g, potassium clavulanate 0.05-5 g, sodium citrate 10-5000 mg, and citric acid 20-1200 mg.

Formulation 269: methicillin sodium 0.5-4 g, potassium clavulanate 0.05-5 g, sodium citrate 10-5000 mg, and citric acid 20-1200 mg.

Formulation 270: nafcillin sodium 0.5-4 g, potassium clavulanate 0.05-5 g, sodium citrate 10-5000 mg, and citric acid 20-1200 mg.

Formulation 271: oxacillin sodium 0.5-4 g, potassium clavulanate 0.05-5 g, sodium citrate 10-5000 mg, and citric acid 20-1200 mg.

Formulation 272: cloxacillin sodium 0.5-4 g, potassium clavulanate 0.05-5 g, sodium citrate 10-5000 mg, and citric acid 20-1200 mg.

Formulation 273: dicloxacillin sodium 0.5-4 g, potassium clavulanate 0.05-5 g, sodium citrate 10-5000 mg, and citric acid 20-1200 mg.

Formulation 274: flucloxacillin sodium 0.5-4 g, potassium clavulanate 0.05-5 g, sodium citrate 10-5000 mg, and citric acid 20-1200 mg.

Formulation 275: mecillinam sodium 0.5-4 g, potassium clavulanate 0.05-5 g, sodium citrate 10-5000 mg, and citric acid 20-1200 mg.

Formulation 276: adicillin sodium 0.5-4 g, potassium clavulanate 0.05-5 g, sodium citrate 10-2000 mg, and citric acid 20-1200 mg.

Formulation 277: ampicillin sodium 0.5-4 g, potassium clavulanate 0.05-5 g, sodium citrate 10-5000 mg, and citric acid 20-1200 mg.

Formulation 278: amoxicillin sodium 0.5-4 g, potassium clavulanate 0.05-5 g, sodium citrate 10-5000 mg, and citric acid 20-1200 mg.

Formulation 279: ticarcillin sodium 0.5-4 g, potassium clavulanate 0.05-5 g, sodium citrate 10-5000 mg, and citric acid 20-1200 mg.

Formulation 280: carbenicillin sodium 0.5-4 g, potassium clavulanate 0.05-5 g, sodium citrate 10-5000 mg, and citric acid 20-1200 mg.

Formulation 281: sulbenicillin sodium 0.5-4 g, potassium clavulanate 0.05-5 g, sodium citrate 10-5000 mg, and citric acid 20-1200 mg.

Formulation 282: hetacillin sodium 0.5-4 g, potassium clavulanate 0.05-5 g, sodium citrate 10-5000 mg, and citric acid 20-1200 mg.

Formulation 283: apalcillin sodium 0.5-4 g, potassium clavulanate 0.05-5 g, sodium citrate 10-5000 mg, and citric acid 20-1200 mg.

Formulation 284: mezlocillin sodium 0.5-4 g, potassium clavulanate 0.05-5 g, sodium citrate 10-5000 mg, and citric acid 20-1200 mg.

Formulation 285: temocillin sodium 0.5-4 g, potassium clavulanate 0.05-5 g, sodium citrate 10-5000 mg, and citric acid 20-1200 mg.

Formulation 286: formidacillin sodium 0.5-4 g, potassium clavulanate 0.05-5 g, sodium citrate 10-5000 mg, and citric acid 20-1200 mg.

Formulation 287: aspoxicillin sodium 0.5-4 g, potassium clavulanate 0.05-5 g, sodium citrate 10-5000 mg, and citric acid 20-1200 mg.

Formulation 288: lenampicillin sodium 0.5-4 g, potassium clavulanate 0.05-5 g, sodium citrate 10-5000 mg, and citric acid 20-1200 mg.

Formulation 289: pheneticillin sodium 0.5-4 g, tazobactam sodium 0.05-5 g, amikacin sulfate 0.05-4 g, sodium citrate 10-5000 mg, and citric acid 20-1200 mg.

Formulation 290: propicillin sodium 0.5-4 g, tazobactam sodium 0.05-5 g, amikacin sulfate 0.05-4 g, sodium citrate 10-5000 mg, and citric acid 20-1200 mg.

Formulation 291: azidocillin sodium 0.5-4 g, tazobactam sodium 0.05-5 g, amikacin sulfate 0.05-4 g, sodium citrate 10-5000 mg, and citric acid 20-1200 mg.

Formulation 292: trityl penicillin sodium 0.5-4 g, tazobactam sodium 0.05-5 g, amikacin sulfate 0.05-4 g, sodium citrate 10-5000 mg, and citric acid 20-1200 mg.

Formulation 293: methicillin sodium 0.5-4 g, tazobactam sodium 0.05-5 g, amikacin sulfate 0.05-4 g, sodium citrate 10-5000 mg, and citric acid 20-1200 mg.

Formulation 294: nafcillin sodium 0.5-4 g, tazobactam sodium 0.05-5 g, amikacin sulfate 0.05-4 g, and sodium citrate 0.01-5 g.

Formulation 295: oxacillin sodium 0.5-4 g, tazobactam sodium 0.05-5 g, amikacin sulfate 0.05-4 g, and sodium citrate 0.01-5 g.

Formulation 296: cloxacillin sodium 0.5-4 g, tazobactam sodium 0.05-5 g, amikacin sulfate 0.05-4 g, and sodium citrate 0.01-5 g.

Formulation 297: dicloxacillin sodium 0.5-4 g, tazobactam sodium 0.05-5 g, amikacin sulfate 0.05-4 g, and sodium citrate 0.01-5 g.

Formulation 298: flucloxacillin sodium 0.5-4 g, tazobactam sodium 0.05-5 g, amikacin sulfate 0.05-4 g, and sodium citrate 0.01-5 g.

Formulation 299: mecillinam sodium 0.5-4 g, tazobactam sodium 0.05-5 g, amikacin sulfate 0.05-4 g, and sodium citrate 0.01-5 g.

Formulation 300: adicillin sodium 0.5-4 g, tazobactam sodium 0.05-5 g, amikacin sulfate 0.05-4 g, and sodium citrate 0.01-5 g.

Formulation 301: ampicillin sodium 0.5-4 g, tazobactam sodium 0.05-5 g, amikacin sulfate 0.05-4 g, and sodium citrate 0.01-5 g.

Formulation 302: amoxicillin sodium 0.5-4 g, tazobactam sodium 0.05-5 g, amikacin sulfate 0.05-4 g, and sodium citrate 0.01-5 g.

Formulation 303: ticarcillin sodium 0.5-4 g, tazobactam sodium 0.05-5 g, amikacin sulfate 0.05-4 g, and sodium citrate 0.01-5 g.

Formulation 304: carbenicillin sodium 0.5-4 g, tazobactam sodium 0.05-5 g, amikacin sulfate 0.05-4 g, and sodium citrate 0.01-5 g.

Formulation 305: sulbenicillin sodium 0.5-4 g, tazobactam sodium 0.05-5 g, amikacin sulfate 0.05-4 g, and sodium citrate0.01-5 g.

Formulation 306: hetacillin sodium 0.5-4 g, tazobactam sodium 0.05-5 g, amikacin sulfate 0.05-4 g, and sodium citrate 0.01-5 g.

Formulation 307: apalcillin sodium 0.5-4 g, tazobactam sodium 0.05-5 g, amikacin sulfate 0.05-4 g, and sodium citrate 0.01-5 g.

Formulation 308: mezlocillin sodium 0.5-4 g, tazobactam sodium 0.05-5 g, amikacin sulfate 0.05-4 g, and sodium citrate 0.01-5 g.

Formulation 309: temocillin sodium 0.5-4 g, tazobactam sodium 0.05-5 g, amikacin sulfate 0.05-4 g, and sodium citrate 0.01-5 g.

Formulation 310: formidacillin sodium 0.5-4 g, tazobactam sodium 0.05-5 g, amikacin sulfate 0.05-4 g, and sodium citrate 0.01-5 g.

Formulation 311: aspoxicillin sodium 0.5-4 g, tazobactam sodium 0.05-5 g, amikacin sulfate 0.05-4 g, and sodium citrate 0.01-5 g.

Formulation 312: lenampicillin sodium 0.5-4 g, tazobactam sodium 0.05-5 g, amikacin sulfate 0.05-4 g, and sodium citrate 0.01-5 g.

Formulation 313: pheneticillin sodium 0.5-4 g, tazobactam sodium 0.05-5 g, gentamicin sulfate 0.05-4 g, and sodium citrate 0.01-5 g.

Formulation 314: propicillin sodium 0.5-4 g, tazobactam sodium 0.05-5 g, gentamicin sulfate 0.05-4 g, and sodium citrate 0.01-5 g.

Formulation 315: azidocillin sodium 0.5-4 g, tazobactam sodium 0.05-5 g, gentamicin sulfate 0.05-4 g, and sodium citrate 0.01-5 g.

Formulation 316: trityl penicillin sodium 0.5-4 g, tazobactam sodium 0.05-5 g, gentamicin sulfate 0.05-4 g, sodium citrate1 0-5000 mg, and citric acid 20-1200 mg.

Formulation 317: methicillin sodium 0.5-4 g, tazobactam sodium 0.05-5 g, gentamicin sulfate 0.05-4 g, sodium citrate 10-100 mg, and citric acid 20-1200 mg.

Formulation 318: nafcillin sodium 0.5-4 g, tazobactam sodium 0.05-5 g, gentamicin sulfate 0.05-4 g, and sodium citrate 0.01-5 g.

Formulation 319: oxacillin sodium 0.5-4 g, tazobactam sodium 0.05-5 g, gentamicin sulfate 0.05-4 g, and sodium citrate 0.01-5 g.

Formulation 320: cloxacillin sodium 0.5-4 g, tazobactam sodium 0.05-5 g, gentamicin sulfate 0.05-4 g, and sodium citrate 0.01-5 g.

Formulation 321: dicloxacillin sodium 0.5-4 g, tazobactam sodium 0.05-5 g, gentamicin sulfate 0.05-4 g, and sodium citrate 0.01-5 g.

Formulation 322: flucloxacillin sodium 0.5-4 g, tazobactam sodium 0.05-5 g, gentamicin sulfate 0.05-4 g, and sodium citrate 0.01-5 g.

Formulation 323: mecillinam sodium 0.5-4 g, tazobactam sodium 0.05-5 g, gentamicin sulfate 0.05-4 g, and sodium citrate 0.01-5 g.

Formulation 324: adicillin sodium 0.5-4 g, tazobactam sodium 0.05-5 g, gentamicin sulfate 0.05-4 g, and sodium citrate 0.01-5 g.

Formulation 325: ampicillin sodium 0.5-4 g, tazobactam sodium 0.05-5 g, gentamicin sulfate 0.05-4 g, and sodium citrate 0.01-5 g.

Formulation 326: amoxicillin sodium 0.5-4 g, tazobactam sodium 0.05-5 g, gentamicin sulfate 0.05-4 g, and sodium citrate 0.01-5 g.

Formulation 327: ticarcillin sodium 0.5-4 g, tazobactam sodium 0.05-5 g, gentamicin sulfate 0.05-4 g, and sodium citrate 0.01-5 g.

Formulation 328: carbenicillin sodium 0.5-4 g, tazobactam sodium 0.05-5 g, gentamicin sulfate 0.05-4 g, and sodium citrate 0.01-5 g.

Formulation 329: sulbenicillin sodium 0.5-4 g, tazobactam sodium 0.05-5 g, gentamicin sulfate 0.05-4 g, and sodium citrate 0.01-5 g.

Formulation 330: hetacillin sodium 0.5-4 g, tazobactam sodium 0.05-5 g, gentamicin sulfate 0.05-4 g, and sodium citrate 0.01-5 g.

Formulation 331: apalcillin sodium 0.5-4 g, tazobactam sodium 0.05-5 g, gentamicin sulfate 0.05-4 g, and sodium citrate 0.01-5 g.

Formulation 332: mezlocillin sodium 0.5-4 g, tazobactam sodium 0.05-5 g, gentamicin sulfate 0.05-4 g, and sodium citrate 0.01-5 g.

Formulation 333: temocillin sodium 0.5-4 g, tazobactam sodium 0.05-5 g, gentamicin sulfate 0.05-4 g, and sodium citrate 0.01-5 g.

Formulation 334: formidacillin sodium 0.5-4 g, tazobactam sodium 0.05-5 g, gentamicin sulfate 0.05-4 g, and sodium citrate 0.01-5 g.

Formulation 335: aspoxicillin sodium 0.5-4 g, tazobactam sodium 0.05-5 g, gentamicin sulfate 0.05-4 g, and sodium citrate 0.01-5 g.

Formulation 336: lenampicillin sodium 0.5-4 g, tazobactam sodium 0.05-5 g, gentamicin sulfate 0.05-4 g, and sodium citrate 0.01-5 g.

Formulation 337: pheneticillin sodium 0.5-4 g, tazobactam sodium 0.05-5 g, etimicin sulfate 0.05-4 g, and sodium citrate 0.01-5 g.

Formulation 338: propicillin sodium 0.5-4 g, tazobactam sodium 0.05-5 g, etimicin sulfate 0.05-4 g, and sodium citrate 0.01-5 g.

Formulation 339: azidocillin sodium 0.5-4 g, tazobactam sodium 0.05-5 g, etimicin sulfate 0.05-4 g, and sodium citrate 0.01-5 g.

Formulation 340: trityl penicillin sodium 0.5-4 g, tazobactam sodium 0.05-5 g, etimicin sulfate 0.05-4 g, and sodium citrate 10-5000 mg, and citric acid 20-1200 mg.

Formulation 341: methicillin sodium 0.5-4 g, tazobactam sodium 0.05-5 g, etimicin sulfate 0.05-4 g, and sodium citrate 10-100 mg, and citric acid 20-1200 mg.

Formulation 342: nafcillin sodium 0.5-4 g, tazobactam sodium 0.05-5 g, etimicin sulfate 0.05-4 g, and sodium citrate 0.01-5 g.

Formulation 343: oxacillin sodium 0.5-4 g, tazobactam sodium 0.05-5 g, etimicin sulfate 0.05-4 g, and sodium citrate 0.01-5 g.

Formulation 344: cloxacillin sodium 0.5-4 g, tazobactam sodium 0.05-5 g, etimicin sulfate 0.05-4 g, and sodium citrate 0.01-5 g.

Formulation 345: dicloxacillin sodium 0.5-4 g, tazobactam sodium 0.05-5 g, etimicin sulfate 0.05-4 g, and sodium citrate 0.01-5 g.

Formulation 346: flucloxacillin sodium 0.5-4 g, tazobactam sodium 0.05-5 g, etimicin sulfate 0.05-4 g, and sodium citrate 0.01-5 g.

Formulation 347: mecillinam sodium 0.5-4 g, tazobactam sodium 0.05-5 g, etimicin sulfate 0.05-4 g, and sodium citrate 0.01-5 g.

Formulation 348: adicillin sodium 0.5-4 g, tazobactam sodium 0.05-5 g, etimicin sulfate 0.05-4 g, and sodium citrate 0.01-5 g.

Formulation 349: ampicillin sodium 0.5-4 g, tazobactam sodium 0.05-5 g, etimicin sulfate 0.05-4 g, and sodium citrate 0.01-5 g.

Formulation 350: amoxicillin sodium 0.5-4 g, tazobactam sodium 0.05-5 g, etimicin sulfate 0.05-4 g, and sodium citrate 0.01-5 g.

Formulation 351: ticarcillin sodium 0.5-4 g, tazobactam sodium 0.05-5 g, etimicin sulfate 0.05-4 g, and sodium citrate 0.01-5 g.

Formulation 352: carbenicillin sodium 0.5-4 g, tazobactam sodium 0.05-5 g, etimicin sulfate 0.05-4 g, and sodium citrate 0.01-5 g.

Formulation 353: sulbenicillin sodium 0.5-4 g, tazobactam sodium 0.05-5 g, etimicin sulfate 0.05-4 g, and sodium citrate 0.01-5 g.

Formulation 354: hetacillin sodium 0.5-4 g, tazobactam sodium 0.05-5 g, etimicin sulfate 0.05-4 g, and sodium citrate 0.01-5 g.

Formulation 355: apalcillin sodium 0.5-4 g, tazobactam sodium 0.05-5 g, etimicin sulfate 0.05-4 g, and sodium citrate 0.01-5 g.

Formulation 356: mezlocillin sodium 0.5-4 g, tazobactam sodium 0.05-5 g, etimicin sulfate 0.05-4 g, and sodium citrate 0.01-5 g.

Formulation 357: temocillin sodium 0.5-4 g, tazobactam sodium 0.05-5 g, etimicin sulfate 0.05-4 g, and sodium citrate 0.01-5 g.

Formulation 358: formidacillin sodium 0.5-4 g, tazobactam sodium 0.05-5 g, etimicin sulfate 0.05-4 g, and sodium citrate 0.01-5 g.

Formulation 359: aspoxicillin sodium 0.5-4 g, tazobactam sodium 0.05-5 g, etimicin sulfate 0.05-4 g, and sodium citrate 0.01-5 g.

Formulation 360: lenampicillin sodium 0.5-4 g, tazobactam sodium 0.05-5 g, etimicin sulfate 0.05-4 g, and sodium citrate 0.01-5 g.

Formulation 361: pheneticillin sodium 0.5-4 g, sulbactam sodium 0.05-5 g, amikacin sulfate 0.05-4 g, sodium citrate 10-5000 mg, and citric acid 20-1200 mg.

Formulation 362: propicillin sodium 0.5-4 g, sulbactam sodium 0.05-5 g, amikacin sulfate 0.05-4 g, sodium citrate 10-5000 mg, and citric acid 20-1200 mg.

Formulation 363: azidocillin sodium 0.5-4 g, sulbactam sodium 0.05-5 g, amikacin sulfate 0.05-4 g, sodium citrate 10-5000 mg, and citric acid 20-1200 mg.

Formulation 364: trityl penicillin sodium 0.5-4 g, sulbactam sodium 0.05-5 g, amikacin sulfate 0.05-4 g, sodium citrate 10-5000 mg, and citric acid 20-1200 mg.

Formulation 365: methicillin sodium 0.5-4 g, sulbactam sodium 0.05-5 g, amikacin sulfate 0.05-4 g, sodium citrate 10-5000 mg, and citric acid 20-1200 mg.

Formulation 366: nafcillin sodium 0.5-4 g, sulbactam sodium 0.05-5 g, amikacin sulfate 0.05-4 g, and sodium citrate 0.01-5 g.

Formulation 367: oxacillin sodium 0.5-4 g, sulbactam sodium 0.05-5 g, amikacin sulfate 0.05-4 g, and sodium citrate 0.01-5 g.

Formulation 368: cloxacillin sodium 0.5-4 g, sulbactam sodium 0.05-5 g, amikacin sulfate 0.05-4 g, and sodium citrate 0.01-5 g.

Formulation 369: dicloxacillin sodium 0.5-4 g, sulbactam sodium 0.05-5 g, amikacin sulfate 0.05-4 g, and sodium citrate 0.01-5 g.

Formulation 370: flucloxacillin sodium 0.5-4 g, sulbactam sodium 0.05-5 g, amikacin sulfate 0.05-4 g, and sodium citrate 0.01-5 g.

Formulation 371: mecillinam sodium 0.5-4 g, sulbactam sodium 0.05-5 g, amikacin sulfate 0.05-4 g, and sodium citrate 0.01-5 g.

Formulation 372: adicillin sodium 0.5-4 g, sulbactam sodium 0.05-5 g, amikacin sulfate 0.05-4 g, and sodium citrate 0.01-5 g.

Formulation 373: ampicillin sodium 0.5-4 g, sulbactam sodium 0.05-5 g, amikacin sulfate 0.05-4 g, and sodium citrate 0.01-5 g.

Formulation 374: amoxicillin sodium 0.5-4 g, sulbactam sodium 0.05-5 g, amikacin sulfate 0.05-4 g, and sodium citrate 0.01-5 g.

Formulation 375: ticarcillin sodium 0.5-4 g, sulbactam sodium 0.05-5 g, amikacin sulfate 0.05-4 g, and sodium citrate 0.01-5 g.

Formulation 376: carbenicillin sodium 0.5-4 g, sulbactam sodium 0.05-5 g, amikacin sulfate 0.05-4 g, and sodium citrate 0.01-5 g.

Formulation 377: sulbenicillin sodium 0.5-4 g, sulbactam sodium 0.05-5 g, amikacin sulfate 0.05-4 g, and sodium citrate 0.01-5 g.

Formulation 378: hetacillin sodium 0.5-4 g, sulbactam sodium 0.05-5 g, amikacin sulfate 0.05-4 g, and sodium citrate 0.01-5 g.

Formulation 379: apalcillin sodium 0.5-4 g, sulbactam sodium 0.05-5 g, amikacin sulfate 0.05-4 g, and sodium citrate 0.01-5 g.

Formulation 380: mezlocillin sodium 0.5-4 g, sulbactam sodium 0.05-5 g, amikacin sulfate 0.05-4 g, and sodium citrate 0.01-5 g.

Formulation 381: temocillin sodium 0.5-4 g, sulbactam sodium 0.05-5 g, amikacin sulfate 0.05-4 g, and sodium citrate 0.01-5 g.

Formulation 382: formidacillin sodium 0.5-4 g, sulbactam sodium 0.05-5 g, amikacin sulfate 0.05-4 g, and sodium citrate 0.01-5 g.

Formulation 383: aspoxicillin sodium 0.5-4 g, sulbactam sodium 0.05-5 g, amikacin sulfate 0.05-4 g, and sodium citrate 0.01-5 g.

Formulation 384: lenampicillin sodium 0.5-4 g, sulbactam sodium 0.05-5 g, amikacin sulfate 0.05-4 g, and sodium citrate 0.01-5 g.

Formulation 385: pheneticillin sodium 0.5-4 g, sulbactam sodium 0.05-5 g, gentamicin sulfate 0.05-4 g, and sodium citrate 0.01-5 g.

Formulation 386: propicillin sodium 0.5-4 g, sulbactam sodium 0.05-5 g, gentamicin sulfate 0.05-4 g, and sodium citrate 0.01-5 g.

Formulation 387: azidocillin sodium 0.5-4 g, sulbactam sodium 0.05-5 g, gentamicin sulfate 0.05-4 g, and sodium citrate 0.01-5 g.

Formulation 388: trityl penicillin sodium 0.5-4 g, sulbactam sodium 0.05-5 g, gentamicin sulfate 0.05-4 g, sodium citrate 10-5000 mg, and citric acid 20-1200 mg.

Formulation 389: methicillin sodium 0.5-4 g, sulbactam sodium 0.05-5 g, gentamicin sulfate 0.05-4 g, sodium citrate 10-5000 mg, and citric acid 20-1200 mg.

Formulation 390: nafcillin sodium 0.5-4 g, sulbactam sodium 0.05-5 g, gentamicin sulfate 0.05-4 g, and sodium citrate 0.01-5 g.

Formulation 391: oxacillin sodium 0.5-4 g, sulbactam sodium 0.05-5 g, gentamicin sulfate 0.05-4 g, and sodium citrate 0.01-5 g.

Formulation 392: cloxacillin sodium 0.5-4 g, sulbactam sodium 0.05-5 g, gentamicin sulfate 0.05-4 g, and sodium citrate 0.01-5 g.

Formulation 393: dicloxacillin sodium 0.5-4 g, sulbactam sodium 0.05-5 g, gentamicin sulfate 0.05-4 g, and sodium citrate 0.01-5 g.

Formulation 394: flucloxacillin sodium 0.5-4 g, sulbactam sodium 0.05-5 g, gentamicin sulfate 0.05-4 g, and sodium citrate 0.01-5 g.

Formulation 395: mecillinam sodium 0.5-4 g, sulbactam sodium 0.05-5 g, gentamicin sulfate 0.05-4 g, and sodium citrate 0.01-5 g.

Formulation 396: adicillin sodium 0.5-4 g, sulbactam sodium 0.05-5 g, gentamicin sulfate 0.05-4 g, and sodium citrate 0.01-5 g.

Formulation 397: ampicillin sodium 0.5-4 g, sulbactam sodium 0.05-5 g, gentamicin sulfate 0.05-4 g, and sodium citrate 0.01-5 g.

Formulation 398: amoxicillin sodium 0.5-4 g, sulbactam sodium 0.05-5 g, gentamicin sulfate 0.05-4 g, and sodium citrate 0.01-5 g.

Formulation 399: ticarcillin sodium 0.5-4 g, sulbactam sodium 0.05-5 g, gentamicin sulfate 0.05-4 g, and sodium citrate 0.01-5 g.

Formulation 400: carbenicillin sodium 0.5-4 g, sulbactam sodium 0.05-5 g, gentamicin sulfate 0.05-4 g, and sodium citrate 0.01-5 g.

Formulation 401: sulbenicillin sodium 0.5-4 g, sulbactam sodium 0.05-5 g, gentamicin sulfate 0.05-4 g, and sodium citrate 0.01-5 g.

Formulation 402: hetacillin sodium 0.5-4 g, sulbactam sodium 0.05-5 g, gentamicin sulfate 0.05-4 g, and sodium citrate 0.01-5 g.

Formulation 403: apalcillin sodium 0.5-4 g, sulbactam sodium 0.05-5 g, gentamicin sulfate 0.05-4 g, and sodium citrate 0.01-5 g.

Formulation 404: mezlocillin sodium 0.5-4 g, sulbactam sodium 0.05-5 g, gentamicin sulfate 0.05-4 g, and sodium citrate 0.01-5 g.

Formulation 405: temocillin sodium 0.5-4 g, sulbactam sodium 0.05-5 g, gentamicin sulfate 0.05-4 g, and sodium citrate 0.01-5 g.

Formulation 406: formidacillin sodium 0.5-4 g, sulbactam sodium 0.05-5 g, gentamicin sulfate 0.05-4 g, and sodium citrate 0.01-5 g.

Formulation 407: aspoxicillin sodium 0.5-4 g, sulbactam sodium 0.05-5 g, gentamicin sulfate 0.05-4 g, and sodium citrate 0.01-5 g.

Formulation 408: lenampicillin sodium 0.5-4 g, sulbactam sodium 0.05-5 g, gentamicin sulfate 0.05-4 g, and sodium citrate 0.01-5 g.

Formulation 409: pheneticillin sodium 0.5-4 g, sulbactam sodium 0.05-5 g, etimicin sulfate 0.05-4 g, and sodium citrate 0.01-5 g.

Formulation 410: propicillin sodium 0.5-4 g, sulbactam sodium 0.05-5 g, etimicin sulfate 0.05-4 g, and sodium citrate 0.01-5 g.

Formulation 411: azidocillin sodium 0.5-4 g, sulbactam sodium 0.05-5 g, etimicin sulfate 0.05-4 g, and sodium citrate 0.01-5 g.

Formulation 412: trityl penicillin sodium 0.5-4 g, sulbactam sodium 0.05-5 g, etimicin sulfate 0.05-4 g, and sodium citrate 0.01-5 g.

Formulation 413: methicillin sodium 0.5-4 g, sulbactam sodium 0.05-5 g, etimicin sulfate 0.05-4 g, and sodium citrate 0.01-5 g.

Formulation 414: nafcillin sodium 0.5-4 g, sulbactam sodium 0.05-5 g, etimicin sulfate 0.05-4 g, and sodium citrate 0.01-5 g.

Formulation 415: oxacillin sodium 0.5-4 g, sulbactam sodium 0.05-5 g, etimicin sulfate 0.05-4 g, and sodium citrate 0.01-5 g.

Formulation 416: cloxacillin sodium 0.5-4 g, sulbactam sodium 0.05-5 g, etimicin sulfate 0.05-4 g, and sodium citrate 0.01-5 g.

Formulation 417: dicloxacillin sodium 0.5-4 g, sulbactam sodium 0.05-5 g, etimicin sulfate 0.05-4 g, and sodium citrate 0.01-5 g.

Formulation 418: flucloxacillin sodium 0.5-4 g, sulbactam sodium 0.05-5 g, etimicin sulfate 0.05-4 g, and sodium citrate 0.01-5 g.

Formulation 419: mecillinam sodium 0.5-4 g, sulbactam sodium 0.05-5 g, etimicin sulfate 0.05-4 g, and sodium citrate 0.01-5 g.

Formulation 420: adicillin sodium 0.5-4 g, sulbactam sodium 0.05-5 g, etimicin sulfate 0.05-4 g, and sodium citrate 0.01-5 g.

Formulation 421: ampicillin sodium 0.5-4 g, sulbactam sodium 0.05-5 g, etimicin sulfate 0.05-4 g, and sodium citrate 0.01-5 g.

Formulation 422: amoxicillin sodium 0.5-4 g, sulbactam sodium 0.05-5 g, etimicin sulfate 0.05-4 g, and sodium citrate 0.01-5 g.

Formulation 423: ticarcillin sodium 0.5-4 g, sulbactam sodium 0.05-5 g, etimicin sulfate 0.05-4 g, and sodium citrate 0.01-5 g.

Formulation 424: carbenicillin sodium 0.5-4 g, sulbactam sodium 0.05-5 g, etimicin sulfate 0.05-4 g, and sodium citrate 0.01-5 g.

Formulation 425: sulbenicillin sodium 0.5-4 g, sulbactam sodium 0.05-5 g, etimicin sulfate 0.05-4 g, and sodium citrate 0.01-5 g.

Formulation 426: hetacillin sodium 0.5-4 g, sulbactam sodium 0.05-5 g, etimicin sulfate 0.05-4 g, and sodium citrate 0.01-5 g.

Formulation 427: apalcillin sodium 0.5-4 g, sulbactam sodium 0.05-5 g, etimicin sulfate 0.05-4 g, and sodium citrate 0.01-5 g.

Formulation 428: mezlocillin sodium 0.5-4 g, sulbactam sodium 0.05-5 g, etimicin sulfate 0.05-4 g, and sodium citrate 0.01-5 g.

Formulation 429: temocillin sodium 0.5-4 g, sulbactam sodium 0.05-5 g, etimicin sulfate 0.05-4 g, and sodium citrate 0.01-5 g.

Formulation 430: formidacillin sodium 0.5-4 g, sulbactam sodium 0.05-5 g, etimicin sulfate 0.05-4 g, and sodium citrate 0.01-5 g.

Formulation 431: aspoxicillin sodium 0.5-4 g, sulbactam sodium 0.05-5 g, etimicin sulfate 0.05-4 g, and sodium citrate 0.01-5 g.

Formulation 432: lenampicillin sodium 0.5-4 g, sulbactam sodium 0.05-5 g, etimicin sulfate 0.05-4 g, and sodium citrate 0.01-5 g.

An iso-osmotic solution may be used to prepare a parenteral solution of the pharmaceutical composition described in this invention. The iso-osmotic solution includes but is not limited to a glucose solution, a fructose solution, and normal saline. The unit dose range of glucose, fructose, or sodium chloride is between 0.1 g and 20 g; and the concentration range in the parenteral solution is between 0.1 % and 10% w/w.

Different formulations (vide supra) were prepared by selecting gentamicin, amikacin, or etimicin with one of four β-lactam antibiotic and one of two β-lactamase inhibitors.

Research on stability and intercomponent compatibility of three dosage forms and preservation methods was carried out. The results shows that in solution preparations prepared by the following three ways: 1) used immediately after preparation; 2) prepared and cryopreserved, then thawed, and then added an aminoglycoside antibiotic prior to use; and 3) prepared as solution, freeze-dried, and preserved at low temperature, then reconstituted as a solution, and then added an aminoglycoside antibiotic prior to use; the contents of the β-lactam antibiotic, β-lactamase inhibitor and aminoglycoside antibiotic were all maintained at levels higher than 90%, and on some cases higher than 95%, which meets technical requirements for clinically applied multi-drug mixtures.

The pharmaceutical composition provides patients and doctors a choice of simultaneous use of β-lactam antibiotics and aminoglycoside antibiotics, and a more effective treatment regimen. More importantly, due to the potential avoidance of drug resistance caused by use of single type of antibiotics, the pharmaceutical composition disclosed herein is more likely to prevent first-time-treatment failure.

The solution formulation of the pharmaceutical composition prepared for microbial control can be used as a parenteral solution, eye drops, nose drops, ear drops, genital duct drops, wash, or a solution for external use. The administration of the solution formulation of the pharmaceutical composition set forth herein as anti-microbial infection drug is preferably by intravenous injection.

The solution formulation of the pharmaceutical composition may be prepared immediately prior to use; or it may be prepared as solution formulation, then sealed, then preserved in a frozen state, and then thawed at room temperature prior to use.

The pharmaceutical composition may be prepared as a solution, injectable powder, or freeze-dried injectable powder and preserved with cold-storage, and re-dissolved into a liquid solution with injectable fluid immediately prior to use.

Still in other aspects of the invention, provided is a method for the preparation of a lyophilized powder of the pharmaceutical composition described herein, comprising: (a) dissolving a β-lactam antibiotic, and other components of the pharmaceutical composition (e.g., the components identified in Formulations 1 to 432 above) in injectable water, or in 2.5% injectable fructose aqueous solution, or in 5% normal saline; (b) adjusting the pH value to between 6 and 6.75; and sub-packaging the resultant solution in containers as unit-dose fluids; (c) placing the unit-dose fluids in a freeze-drier (lyophilizer); adjusting the temperature of the freeze drier to minus 35°C, and pumping the atmosphere of the freeze drier to below 40 Pa; (d) adjusting the temperature to between 3 and 5°C, and removing water completely; (e) adjusting the temperature of the freeze drier to 40-50°C and drying the obtained freeze-dried injectable powder; (f) blowing dry nitrogen into the freeze-drier, and sealing the bottles with sterile seal-capping; and (g) storing below 5°C in the dark.

The amounts of β-lactam antibiotics and β-lactamase inhibitor were determined using C18 reverse-phase LC and UV-VIS detector (Tianjin Hemay BioTech Co., Ltd., analysis method No. HM-K-03). The content of aminoglycoside antibiotics was measured using reverse-phase HPLC and evaporation-light-scattering detector (ELSD detector) (Tianjin Hemay BioTech Co., Ltd., analysis method No. HM-K-08). The amount of antibiotic component at each time point was expressed as a percentage with respect to the amount at the initial time (herein defined as 100%). The relative amount of each antibiotic component in the composition at each time point was expressed as peak area ratio with respect to the corresponding peak area of an external standard.

### Example 1

Pharmaceutical composition of cefoperazone sodium and gentamicin.

Preparation

Cefoperazone sodium (4 g) was dissolved in 200 mL of injectable water, and gentamicin parenteral solution (80 mg in 2 mL of injectable water) was added. Upon mixing, a large amount of white solid precipitated out of the solution immediately.

### Example 2

Pharmaceutical composition of cefoperazone sodium, gentamicin, and buffer.

40 mg of cefoperazone sodium was dissolved in 2 mL of various buffer solutions with different pH value and concentration, and then 20 µL of gentamicin parenteral solution with concentration of 80 mg/2 mL was added. The mixture was ultrasonicated for 5 minutes and clarity observed. Clear solutions were obtained, and there was no precipitate generated when the pH value of buffer solution was above 6. The type of buffer solution used had little effect on the results. The results are shown in the table below.

| Buffer solution | pH of buffer solutions | Concentration of buffer solutions (mM) | Results |
|---|---|---|---|
| Citric acid/sodium citrate | 5 | 10 | *** |
| Citric acid/sodium citrate | 5.5 | 10 | ** |
| Citric acid/sodium citrate | 6 | 10 | * |
| Citric acid/sodium citrate | 6.5 | 10 | OK |
| Citric acid/sodium citrate | 7.0 | 10 | OK |
| Citric acid/sodium citrate | 7.5 | 10 | OK |
| Citric acid/sodium citrate | 6 | 20 | OK |
| Acetic acid/sodium acetate | 6.5 | 20 | OK |
| Phosphoric acid/ disodium hydrogen phosphate | 6.5 | 20 | OK |
| Arginine/arginine sodium | 6.5 | 20 | OK |
| *the solution turned slightly turbid, **the solution turned turbid, ***the solution turned fairly turbid, OK: clear solution | | | |

### Example 3

Pharmaceutical composition of cefoperazone sodium, sulbactam sodium, gentamicin, and buffer.

40 mg of cefoperazone sodium and 5 mg of sulbactam sodium were dissolved in 2 mL of various buffer solutions with different pH values and concentrations. Then, 20 µL of gentamicin parenteral solution having a concentration of 40 mg/mL was added. The mixture was ultrasonicated for 5 minutes and clarity observed. Clear solutions were obtained, and there was no precipitate generated when the pH value of buffer solution was above 6 and the concentration was higher than 20 mM. The type of buffer solution had little effect on the results. The results are shown in the table below.

| Buffer solution | pH of buffer solution | Concentration of buffer solutions (mM) | Results |
|---|---|---|---|
| Citric acid/sodium citrate | 5.5 | 10 | ** |
| Citric acid/sodium citrate | 6 | 10 | * |
| Citric acid/sodium citrate | 6.5 | 10 | OK |
| Citric acid/sodium citrate | 7.0 | 10 | OK |
| Citric acid/sodium citrate | 7.5 | 10 | OK |
| Citric acid/sodium citrate | 6 | 20 | OK |
| *the solution turned slightly turbid, **the solution turned turbid, ***the solution turned fairly turbid, OK: clear solution | | | |

### Example 4

Pharmaceutical composition of cefoperazone sodium, sulbactam sodium, gentamicin, sodium citrate, and citric acid.

Preparation

Sodium citrate (0.20 g) and citric acid (as much as necessary) were dissolved in 200 mL of injectable water to give a solution with the pH value of 6.75. Cefoperazone sodium (4 g) and sulbactam sodium (0.5 g) were then added, and the mixture was shaken. Gentamicin parenteral solution (80 mg in 2 mL) was added dropwise, and a small amount of white floc had precipitated out. After 15 min of ultrasonication, the white floc had dissolved to give a clear solution. The solution was packaged in a bottle or a bag and stored at room temperature. Observation of the appearance of the precipitate was conducted at 0, 1, 2, 4, and 6 hours. The amounts of cefoperazone sodium and sulbactam sodium were determined by sampling at the above-mentioned time points, and the results are shown below:

| Time (h) | 0 | 1 | 2 | 4 | 6 |
|---|---|---|---|---|---|
| Cefoperazone sodium | 101.4 | 98.66 | 98.79 | 101.5 | 107.4 |
| Sulbactam sodium | 101.4 | 101.4 | 101.5 | 109.7 | 103.2 |

### Example 5

Pharmaceutical composition of cefoperazone sodium, sulbactam sodium, amikacin sulfate, and sodium citrate.

Preparation

Cefoperazone sodium (4 g), sulbactam sodium (0.5 g) and sodium citrate (0.20 g) were dissolved in 30 mL of injectable water; the pH value was adjusted to 6.75 with citric acid or sodium hydroxide aqueous solution. After 5 min of ultrasonication, a clear solution was obtained. The resultant solution was filtered (through a 0.2 µm filter), then loaded into containers and placed in a freeze-drier. The temperature of the freeze drier was adjusted to minus 35°C, and the vacuum of the freeze drier was pumped to below 30 Pa. Then the temperature was adjusted to 3°C, and water was removed completely. The resultant freeze-dried injectable powder was dried at 40°C. Dry nitrogen was blown in, and freeze-dried injectable powder of amikacin sulfate (500 mg) was added. The containers were sealed with sterile seal-caps, and stored in a refrigerator at 0°C. After 7 days, the freeze-dried injectable powder was dissolved in 100 mL of injectable water. Observation of the appearance of the precipitate was conducted at 0, 1, 2, 4, and 6 hours. The amounts of cefoperazone sodium, sulbactam sodium, and amikacin were determined by sampling at the above-mentioned time points, and the results are shown below:

| Time (h) | 0 | 1 | 2 | 4 | 6 |
|---|---|---|---|---|---|
| Cefoperazone sodium | 101.4 | 98.66 | 98.79 | 101.5 | 107.4 |
| Sulbactam sodium | 101.4 | 101.4 | 101.5 | 109.7 | 103.2 |
| Amikacin | 98.98 | 98.73 | 98.52 | 98.41 | 98.76 |

### Example 6

Pharmaceutical composition of cefoperazone sodium, sulbactam sodium, etimicin sulfate, and sodium citrate/citric acid.

Preparation

Cefoperazone sodium (4 g), sulbactam sodium (1 g) and sodium citrate (0.20 g) were dissolved in 30 mL of injectable water. The pH value was adjusted to 6.75 with citric acid or sodium hydroxide aqueous solution. 30 mL of the resultant solution was filtered (through a 0.2 µm filter) and then loaded into containers and placed into a freeze-drier. The temperature of the freeze drier was adjusted to minus 35°C, and the vacuum of the freeze drier was pumped to below 30 Pa. The temperature was adjusted to 3°C, and water was removed completely. The resultant freeze-dried injectable powder was dried at 40°C. Dry nitrogen was blown in, and freeze-dried injectable powder of etimicin sulfate (200 mg) was added and the containers were sealed with sterile seal-caps. The containers were stored in refrigerator at 0°C. After 7 days, the freeze-dried injectable powder was dissolved in 100 mL of injectable water. After 10 min of ultrasonication, a clear solution was obtained. Observation of the appearance of the precipitate was conducted at 0, 1, 2, 4, 6 and 8 hours. The amounts of cefoperazone sodium, sulbactam sodium, and etimicin were determined by sampling at the above-mentioned time points, and the results are shown below:

| Time (h) | 0 | 1 | 2 | 4 | 6 | 8 |
|---|---|---|---|---|---|---|
| Cefoperazone sodium | 110.4 | 99.48 | 101.3 | 98.66 | 98.02 | 99.30 |
| Sulbactam sodium | 99.62 | 100.4 | 100.2 | 100.2 | NA | NA |
| Etimicin | 108.9 | 100.2 | 100.5 | 100.4 | 100.4 | 97.50 |
| NA: undetermined content | | | | | | |

### Example 7

Pharmaceutical composition of ceftriaxone sodium, sulbactam sodium, gentamicin sulfate, sodium citrate, and citric acid.

Preparation

Citric acid and sodium citrate (0.20 g) were dissolved in 200 mL of injectable water to give a solution with the pH value of 6.75. Ceftriaxone sodium (4 g) and sulbactam sodium (1 g) were added, and the mixture was shaken. Gentamicin sulfate, 80 mg/2 mL parenteral solution, was added dropwise, and a small amount of white floc had formed. After 15 min of ultrasonication, the white floc had dissolved to give a clear solution. The solution was sealed and stored at room temperature (22°C). Observation of the appearance of the solution was conducted at 0, 1, 2, 4, 6 and 8 hours. The amounts of ceftriaxone sodium and sulbactam sodium were determined by sampling at the above-mentioned time points, and the results are shown below:

| Time (h) | 0 | 1 | 2 | 4 | 6 | 8 |
|---|---|---|---|---|---|---|
| Ceftriaxone sodium | 97.01 | 100.3 | 96.40 | 99.01 | 98.91 | 99.53 |
| Sulbactam sodium | 96.84 | 94.43 | 93.35 | 93.23 | 92.13 | 93.38 |

### Example 8

Pharmaceutical composition of ceftriaxone sodium, tazobactam sodium, gentamicin sulfate, sodium citrate, and citric acid.

Preparation

Sodium citrate (0.20 g) was dissolved in 200 mL of 2.5% aqueous fructose solution and citric acid was added in an amount sufficient to lower the pH value to 6.75. Ceftriaxone sodium (4 g) and tazobactam sodium (1 g) were added, and the mixture was shaken. Gentamicin sulfate parenteral solution (80 mg in 2 mL) was added dropwise, and a small amount of white floc has precipitated out. After 15 min of ultrasonication, the white floc had dissolved to give a clear solution.

100 mL of the resultant solution was filtered (through a 0.2 µm filter) into a container and placed into the freeze-drier. The freeze-drier temperature was adjusted to minus 35°C, and the atmosphere of the freeze-drier was pumped to 30 Pa. After removal of water at 3°C, the temperature of the freeze-drier was adjusted to 40°C to obtain freeze-dried injectable powder. Dry nitrogen was charged and the container was sealed with a sterile cover, and stored in a refrigerator at 0°C. After 7 days, the freeze-dried injectable powder was prepared as solution by mixing with 100 mL of injectable water. Observation of the appearance of precipitate was conducted at 0, 1, 2, 4, 6 and 8 hours. The amounts of ceftriaxone sodium and tazobactam sodium in the solution were determined, and the results are shown below:

| Time (h) | 0 | 1 | 2 | 4 | 6 | 8 |
|---|---|---|---|---|---|---|
| Ceftriaxone sodium | 98.75 | 100.1 | 99.97 | 98.87 | 99.27 | 100.5 |
| Tazobactam sodium | 102.2 | 97.53 | 94.16 | 94.58 | 95.45 | 94.44 |

### Example 9

Pharmaceutical composition of ceftriaxone sodium, etimicin sulfate, sodium citrate, and citric acid.

Preparation

Sodium citrate (0.20 g) was dissolved in 200 mL injectable water and citric acid was added in an amount sufficient to lower the pH value to 6.75. Ceftriaxone sodium (4 g) were added, the mixture was then shaken. 100 mL of the resultant solution was filtered (though a 0.2µm filter) into a container and placed into a freeze-drier. The freeze-drier temperature was adjusted to minus 35°C and the atmosphere of the freeze-dried was pumped to 30 Pa. After removal of water at 3°C, the temperature of the freeze-drier was adjusted to 40°C to obtain freeze-dried injectable powder. Dry nitrogen was charged and the container was sealed with a sterile cover, and stored in a refrigerator at 0°C. After 7 days, the freeze-dried injectable powder was dissolved by mixing with 100 mL of 5% injectable fructose aqueous solution. Observation of the appearance of precipitate was conducted at 0, 1, 2, 4, 6 and 8 hours. The amount of ceftriaxone sodium in the solution were determined, and the results are shown below:

| Time (h) | 0 | 1 | 2 | 4 | 6 | 8 |
|---|---|---|---|---|---|---|
| Ceftriaxone sodium | 97.27 | 99.38 | 99.95 | 97.81 | 99.03 | 98.51 |

### Example 10

Pharmaceutical composition of mezlocillin sodium, etimicin sulfate, sodium citrate, and citric acid.

Preparation

Sodium citrate (0.20 g) was dissolved in 200 mL injectable water and citric acid was added in an amount sufficient to lower the pH value to 6.75. Mezlocillin sodium (2 g) and sulbactam sodium (0.5 g) were added, and the mixture was then shaken. Etimicin sulfate (200 mg in 2 mL) was added with agitation, and small amount of white floc had precipitated out. After 15 min of ultrasonication, the white floc had dissolved to give a clear solution (22°C). The solution was sealed and stored at room temperature. Observation of the appearance of the precipitate was conducted at 0, 1, 2, 4, 6 and 8 hours. The amount of mezlocillin sodium, sulbactam sodium, and etimicin sulfate in the solution were determined, and the results are shown below

| Time (h) | 0 | 1 | 2 | 4 | 6 | 8 |
|---|---|---|---|---|---|---|
| Mezlocillin | 96.48 | 95.90 | 71.70 | 91.78 | 93.08 | 97.69 |
| Sulbactam sodium | 98.07 | 98.13 | 72.96 | 95.16 | 99.65 | 104.9 |
| Etimicin sulfate | 100.1 | 100.6 | 99.79 | NA | NA | NA |
| NA: undetermined content | | | | | | |

### Example 11

Pharmaceutical composition of mezlocillin sodium, sulbactam sodium, etimicin sulfate, sodium citrate, and citric acid.

Preparation

Sodium citrate (0.20 g) was dissolved in 200 mL injectable water and citric acid was added in an amount sufficient to lower the pH value to 6.75. Mezlocillin sodium (2 g) and sulbactam sodium (1 g) were added, the mixture was then shaken. 100 mL of the resultant solution was filtrated into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of 5% injectable fructose aqueous solution. 200 mg of etimicin sulfate was added and the mixture was agitated. A small amount of white floc had precipitated out. After 15 min of ultrasonication, the white floc had dissolved to give a clear solution. Observation of the appearance of the precipitate was conducted at 0, 1, 2, 4, 6 and 8 hours. The content of mezlocillin sodium, sulbactam sodium, and etimicin sulfate in the solution was determined, and the results are shown below:

| Time (h) | 0 | 1 | 2 | 4 | 6 | 8 |
|---|---|---|---|---|---|---|
| Mezlocillin sodium | 116.6 | 94.83 | 91.86 | 91.96 | 91.52 | 98.56 |
| Sulbactam sodium | 114.3 | 95.83 | 93.75 | 93.78 | 95.65 | 101.0 |
| Etimicin sulfate | 100.1 | 100.4 | 99.31 | 99.56 | 99.61 | 99.25 |

### Example 12

Pharmaceutical composition of cefodizime sodium, sodium citrate and citric acid.

Preparation

Sodium citrate (0.20 g) was dissolved in 200 mL injectable water and citric acid was added in an amount sufficient to lower the pH value to 6.75. Cefodizime sodium (4 g) was added, the mixture was then shaken. 100 mL of the resultant solution was filtered (through a 0.2µm filter) into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of 5% injectable fructose aqueous solution. 200 mg of etimicin sulfate was added and the mixture was agitated. A small amount of white floc had precipitated out. After 15 min of ultrasonication, the white floc had dissolved to give a clear solution. Observation of the appearance of the precipitate was conducted at 0, 1, 2, 4, 6 and 8 hours. The content of cefodizime sodium in the solution was determined, and the results are shown below:

| Time (h) | 0 | 1 | 2 | 4 | 6 | 8 |
|---|---|---|---|---|---|---|
| Cefodizime sodium | 99.01 | 103.8 | 98.33 | 98.99 | 97.89 | 92.52 |

### Example 13

Pharmaceutical composition of mezlocillin sodium, etimicin sulfate, sodium citrate and citric acid.

Preparation

Sodium citrate (0.20 g) was dissolved in 200 mL injectable water and citric acid was added in an amount sufficient to lower the pH value to 6. Mezlocillin sodium (4 g) was added, the mixture was then shaken. 100 mL of the resultant solution was filtered (through a 0.2µm filter) into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C

After 7 days, the resultant freeze-dried injectable powder was reconstituted by mixing with 100 mL of 5% injectable fructose aqueous solution. 200 mg of etimicin sulfate was added and the mixture was agitated. A small amount of white floc had precipitated out. After 15 min of ultrasonication, the white floc had dissolved to give a clear solution. Observation of the appearance of the precipitate was conducted at 0, 1, 2, 4, 6 and 8 hours. The content of mezlocillin sodium in the solution was determined, and the results are shown below:

| Time (h) | 0 | 1 | 2 | 4 | 6 | 8 |
|---|---|---|---|---|---|---|
| | | | | | | |
| Mezlocillin sodium | 95.05 | 96.63 | 89.33 | 98.72 | 92.73 | 92.51 |

### Example 14

Pharmaceutical composition of cefoperazone sodium, sulbactam sodium, gentamicin sulfate, sodium citrate, and citric acid.

Preparation

Cefoperazone sodium (4 g), sulbactam sodium (2 g), and sodium citrate (0.5 g) were dissolved in 10 mL of injectable water. The pH value of the solution was adjusted to 6.75 with citric acid. After 10 min of ultrasonication, a clear solution was obtained. 100 mL of the resultant solution was filtered (through a 0.2µm filter) into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 16 mg of gentamicin sulfate. After 10 minutes of ultrasonication, a clear solution was obtained. Observation of the appearance of the precipitate was conducted at 0, 1, 2, 4, and 6 hours. The content of cefoperazone sodium and sulbactam sodium in the solution was determined, and the results are shown below:

| Time (h) | 0 | 1 | 2 | 4 | 6 |
|---|---|---|---|---|---|
| Cefoperazone sodium | 98.42 | 97.39 | 97.46 | 94.70* | 93.02* |
| Sulbactam sodium | 92.44 | 91.27 | 90.78 | 88.38* | 86.86* |
| *the solution turned slightly turbid | | | | | |

### Example 15

Pharmaceutical composition of cefoperazone sodium, sulbactam sodium, gentamicin sulfate, sodium citrate, and citric acid.

Preparation

Cefoperazone sodium (4 g), sulbactam sodium (4 g), and sodium citrate (0.20 g) were dissolved in 10 mL injectable water. The pH value of the solution was adjusted to 6.75 with citric acid. After 10 min of ultrasonication, a clear solution was obtained. The resultant solution was filtered (though 0.2 µm filter) into a container and placed into a freeze-drier. The freeze-drier temperature was adjusted to minus 35°C, and the atmosphere of the freeze-dried was pumped to 30 Pa. After removal of water at 2°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 16 mg of gentamicin sulfate. After 10 minutes of ultrasonication, a clear solution was obtained. Observation of the appearance of the precipitate was conducted at 0, 1, 2, 4, and 6 hours. The content of cefoperazone sodium and sulbactam sodium in the solution was determined, and the results are shown below:

| Time (h) | 0 | 1 | 2 | 4 | 6 |
|---|---|---|---|---|---|
| Cefoperazone sodium | 101.7 | 96.06 | 95.72 | 97.32* | 69.95* |
| Sulbactam sodium | 99.50 | 96.28 | 102.5 | 95.78* | 70.74* |
| *the solution turned slightly turbid | | | | | |

### Example 16

Pharmaceutical composition of cefoperazone sodium, tazobactam sodium, etimicin sulfate, gentamicin sulfate, sodium citrate, and citric acid.

Preparation

Cefoperazone sodium (4 g), tazobactam sodium (1 g), and sodium citrate (0.2 g) were dissolved in 10 mL injectable water. The pH value of the solution was adjusted to 6.75 with citric acid. After 10 min of ultrasonication, a clear solution was obtained. The resultant solution was filtered (though 0.2 µm filter) into a container and placed into a freeze-drier. The freeze-drier temperature was adjusted to minus 35°C, and the atmosphere of the freeze-dried was pumped to 40 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 20 mg of etimicin sulfate. After 10 minutes of ultrasonication, a clear solution was obtained. Observation of the appearance of the precipitate was conducted at 0, 1, 2, 4, 6 and 8 hours. The content of cefoperazone sodium and tazobactam sodium in the solution was determined, and the results are shown below:

| Time (h) | 0 | 1 | 2 | 4 | 6 | 8 |
|---|---|---|---|---|---|---|
| Cefoperazone sodium | 102.9 | 108.6 | 104.6 | 99.50 | 98.91 | 103.4 |
| Tazobactam sodium | 98.77 | 108.2 | 110.8 | 97.39 | 106.3 | 106.3 |

10 mL of the above final solution were separated and to it was added 16 mg of gentamicin sulfate. After 10 minutes of ultrasonication, a clear solution was obtained. Observation of the appearance of the precipitate was conducted at 0, 1, 2, 4, 6 and 8 hours. The content of cefoperazone sodium and tazobactam sodium in the solution was determined, and the results are shown below:

| Time (h) | 0 | 1 | 2 | 4 | 6 | 8 |
|---|---|---|---|---|---|---|
| Cefoperazone sodium | 92.44 | 104.6 | 102.4 | 97.95* | 98.90* | 102.9* |
| Tazobactam sodium | 93.36 | 104.8 | 100.0 | 96.58* | 106.1* | 107.1* |
| *the solution turned slightly turbid | | | | | | |

### Example 17

Pharmaceutical composition of cefoperazone sodium, sulbactam sodium, amikacin sulfate, sodium citrate, and citric acid.

Preparation

Cefoperazone sodium (4 g), sulbactam sodium (1 g), and sodium citrate (0.20 g) were dissolved in 10 mL injectable water. The pH value of the solution was adjusted to 6.75 with citric acid. After 10 min of ultrasonication, a clear solution was obtained. The resultant solution was filtered (though 0.2 µm filter) into a container and placed into a freeze-drier. The freeze-drier temperature was adjusted to minus 35°C, and the atmosphere of the freeze-dried was pumped to 40 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 40 mg amikacin sulfate. After 10 minutes of ultrasonication, a clear solution was obtained. Observation of the appearance of the precipitate was conducted at 0, 1, 2, 4, 6 and 8 hours. The content of cefoperazone sodium, sulbactam sodium and amikacin sulfate in the solution was determined, and the results are shown below:

| Time (h) | 0 | 1 | 2 | 4 | 6 | 8 |
|---|---|---|---|---|---|---|
| Cefoperazone sodium | 92.30 | 94.80 | 94.06 | 94.12 | 93.15 | 94.50 |
| Sulbactam sodium | 105.6 | 112.2 | 112.6 | 118.5 | 123.3 | 127.1 |
| Amikacin sulfate | 99.91 | 99.50 | 98.88 | 99.45 | 98.56 | 99.36 |

### Example 18

Pharmaceutical composition of cefodizime sodium, etimicin sulfate, sodium citrate, and citric acid.

Preparation

Sodium citrate (0.20 g) was dissolved in 200 mL of injectable water. Enough citric acid was added to give a solution having the pH value of 6.75. Cefodizime sodium (1 g) was added, the mixture was shaken to give a solution. Etimicin sulfate (200 mg) was added with agitation. Small amount of white floc had formed. After 15 min of ultrasonication, the floc was dissolved to give a clear solution. The solution was sealed and stored at room temperature. Observation of the appearance of the precipitate was conducted at 0, 1, 2, 4, 6 and 8 hours. The amount of cefodizime sodium in the mixture was determined, and the results are shown below:

| Time (h) | 0 | 1 | 2 | 4 | 6 | 8 |
|---|---|---|---|---|---|---|
| Cefodizime sodium | 99.01 | 103.8 | 98.33 | 98.99 | 97.89 | 92.52 |

### Example 19

Pharmaceutical composition of cefoperazone sodium, sulbactam sodium, gentamicin sulfate, sodium citrate, and citric acid.

Preparation

Sodium citrate (0.20 g) was dissolved in 200 mL of injectable water. Enough citric acid was added to give a solution having the pH value of 6.75. Ceftriaxone sodium (2 g) and sulbactam sodium (2 g) were added, and the mixture was shaken. Gentamicin sulfate (160 mg) was added and the mixture was agitated. A small amount of white floc had formed. After 15 minutes of ultrasonication, a clear solution was obtained. The solution was sealed and stored at room temperature. Observation of the appearance of the precipitate was conducted at 0, 1, 2, 4, 6 and 8 hours. The content of ceftriaxone sodium and sulbactam sodium in the solution was determined, and the results are shown below:

| Time (h) | 0 | 1 | 2 | 4 | 6 | 8 |
|---|---|---|---|---|---|---|
| Ceftriaxone sodium | 97.01 | 100.3 | 96.40 | 99.01 | 98.91 | 99.53 |
| Sulbactam sodium | 96.84 | 94.43 | 93.35 | 93.23 | 92.13 | 93.38 |

### Example 20

Pharmaceutical composition of ceftriaxone sodium, tazobactam sodium, gentamicin sulfate, sodium citrate, and citric acid.

Preparation

Sodium citrate (0.20 g) was dissolved in 200 mL of injectable water. Enough citric acid was added to give a solution having the pH value of 6.75. Ceftriaxone sodium (2 g) and tazobactam sodium (2 g) were added, and the mixture was shaken. Gentamicin sulfate (160 mg) was added and the mixture was agitated. A small amount of white floc had formed. After 15 minutes of ultrasonication, a clear solution was obtained. The solution was sealed and stored at room temperature. Observation of the appearance of the precipitate was conducted at 0, 1, 2, 4, 6 and 8 hours. The content of ceftriaxone sodium and tazobactam sodium in the solution was determined, and the results are shown below

| Time (h) | 0 | 1 | 2 | 4 | 6 | 8 |
|---|---|---|---|---|---|---|
| Ceftriaxone sodium | 98.75 | 100.1 | 99.97 | 98.87 | 99.27 | 100.5 |
| Tazobactam sodium | 102.2 | 97.53 | 94.16 | 94.58 | 95.45 | 94.44 |

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A pharmaceutical composition, comprising: at least one β-lactam antibiotic, at least one β-lactamase inhibitor, and at least one buffer component.

2. The pharmaceutical composition of claim 1, wherein said β-lactam antibiotic is selected from cefalothine, cefaloridine, cefazolin, cefapirin, cefaloglycin, cefalexin, cefadroxil, cefaclor, cefamandole, cefsulodine , cefoperazone, cefuroxime, cefotaxime, ceftizoxime, cefmenoxime, ceftriaxone, cefuzonam, cefixime, ceftazidime, ceftibuten, cefodizime, cephalosporin, cefpirome, cefepime, cefclidin, cefoxitin, cefmetazol, cefbuperazone, cefotetan, latamoxef, flomoxef, loracarbef, pheneticillin, propicillin, azidocillin, trityl penicillin, methicillin, nafcillin, oxacillin, cloxacillin, dicloxacillin, flucloxacillin, mecillinam, adicillin, ampicillin, amoxicillin, ticarcillin, carbenicillin, sulbenicillin, hetacillin, apalcillin, mezlocillin, temocillin, formidacillin, aspoxicillin, lenampicillin, azlocillin, metampicillin, furbenicillin, pivampicillin, atabicillin, or a pharmaceutically acceptable salt or hydrate thereof.

3. The pharmaceutical composition of claim 1, wherein said buffer component is selected from citric acid/citrate, phosphoric acid/phosphate, acetic acid/ acetate, arginine, carbonic acid/carbonate, or tris/HCl.

4. The pharmaceutical composition of claim 3, wherein an effective pH range of said buffer component is between 5.5 and 7.5.

5. The pharmaceutical composition of claim 3, wherein an effective pH range of said buffer component is between 6 and 6.75.

6. The pharmaceutical composition of claim 1, wherein said β-lactamase inhibitor is selected from clavulanic acid, sulbactam, tazobactam, or a pharmaceutically acceptable salt or a hydrate thereof.

7. The pharmaceutical composition of claim 1 or 6, further comprising at least one aminoglycoside antibiotic.

8. The pharmaceutical composition of claim 7, wherein said aminoglycoside antibiotic is selected from etimicin, gentamicin, tobramycin, amikacin, netilmicin, dibekacin, kanamycin, arbekacin, sagamicin, Isepamicin, sisomicin, neomycin, paromomycin, streptomycin, spectinomycin, micronomicin, astromicin, ribostamycin, or a pharmaceutically acceptable salt or a hydrate thereof.

9. The pharmaceutical composition of claim 7, provided in a unit dose formulation, said unit dose comprising between 0.01 g and 5 g of said aminoglycoside antibiotic.

10. The pharmaceutical composition of claim 1, 6, or 7, provided in a unit dose formulation, said unit dose comprising between 0.1 g and 5 g of said β-lactam antibiotic.

11. The pharmaceutical composition of claim 1, 6, or 7, provided in a unit dose formulation, said unit dose comprising between 0.1 g and 5 g of said β-lactam inhibitor.

12. The pharmaceutical composition of claim 1, 6 or 7, further comprising at least one iso-osmotic component.

13. The pharmaceutical composition of claim 13, wherein said iso-osmotic component is glucose, fructose, or sodium chloride.

14. The pharmaceutical composition of claim 1, 6, 7, or 13, provided as a parenteral solution, powder for injection, or lyophilized powder for injection.
